# EUROPEAN PATENT SPECIFICATION

(11) **EP 3 510 049 B1**
(45) Date of publication and mention of the grant of the patent: **08.11.2023**
(21) Application number: 17768082.4
(22) Date of filing: 12.09.2017
(51) Int. Cl.: C07K 16/44

(54) **ACID-ALPHA GLUCOSIDASE VARIANTS AND USES THEREOF**
SAURE ALPHA-GLUCOSIDASE-VARIANTEN UND VERWENDUNGEN DAVON
VARIANTS D'ALPHA-GLUCOSIDASE ACIDE ET LEURS UTILISATIONS

(30) Priority: 12.09.2016 EP 16306149
(43) Date of publication of application: 17.07.2019
(73) Proprietor: Genethon, 91000 Evry (FR); SORBONNE UNIVERSITE, 75006 Paris (FR)
(72) Inventor: MINGOZZI, Federico, 75011 Paris (FR); RONZITTI, Giuseppe, 77300 Fontainebleau (FR); COLELLA, Pasqualina, 80048 S. Anastasia NA (IT); PUZZO, Francesco, Menlo Park 94025, California (US)
(74) Representative: Cabinet Becker et Associés
(86) International application number: PCT/EP2017/072945
(87) International publication number: WO 2018/046775

(56) References cited:
- WO-A1-2012/085622
- WO-A1-2015/192092
- WO-A1-2016/065319
- WO-A2-2013/013017
- US-A1- 2005 281 805
- US-A1- 2013 316 366
- JEFF ZHIQIANG LU ET AL: "Genetic Engineering of a Bifunctional IgG Fusion Protein with Iduronate-2-Sulfatase", BIOCONJUGATE CHEMISTRY,, vol. 21, no. 1, 20 January 2010 (2010-01-20), pages 151-156, XP002692096, ISSN: 1043-1802, DOI: 10.1021/BC900382Q [retrieved on 2009-12-14]

## Description

The present invention relates to variants of acid-alpha glucosidase (GAA) and uses thereof. Said variants are linked to heterogenous signal peptides.

Pompe disease, also known as glycogen storage disease (GSD) type II and acid maltase deficiency, is an autosomal recessive metabolic myopathy caused by a deficiency of the lysosomal enzyme acid alpha-glucosidase (GAA). GAA is an exo-1,4 and 1,6-α-glucosidase that hydrolyzes glycogen to glucose in the lysosome. Deficiency of GAA leads to glycogen accumulation in lysosomes and causes progressive damage to respiratory, cardiac, and skeletal muscle. The disease ranges from a rapidly progressive infantile course that is usually fatal by 1-2 years of age to a more slowly progressive and heterogeneous course that causes significant morbidity and early mortality in children and adults. Hirschhorn RR, The Metabolic and Molecular Bases of Inherited Disease, 3: 3389-3420 (2001, McGraw-Hill); Van der Ploeg and Reuser, Lancet 372: 1342-1351 (2008).

Current human therapy for treating Pompe disease involves administration of recombinant human GAA, otherwise termed enzyme-replacement therapy (ERT). ERT has demonstrated efficacy for severe, infantile GSD II. However the benefit of enzyme therapy is limited by the need for frequent infusions and the development of inhibitor antibodies against recombinant hGAA (Amalfitano, A., et al. (2001) Genet. In Med. 3:132-138). Furthermore, ERT does not correct efficiently the entire body, probably because of a combination of poor biodistribution of the protein following peripheral vein delivery, lack of uptake from several tissues, and high immunogenicity.

As an alternative or adjunct to ERT, the feasibility of gene therapy approaches to treat GSD-II have been investigated (Amalfitano, A., et al. (1999) Proc. Natl. Acad. Sci. USA 96:8861-8866, Ding, E., et al. (2002) Mol. Ther. 5:436-446, Fraites, T. J., et al. (2002) Mol. Ther. 5:571-578, Tsujino, S., et al. (1998) Hum. Gene Ther. 9:1609-1616). However, muscle-directed gene transfer to correct the genetic defect has to face the limitation of the systemic nature of the disease and the fact that muscle expression of a transgene tends to be more immunogenic compared with other tissues.

Doerfler et al., 2016 describe the combined administration of two constructs encoding a human codon-optimized GAA, one under the control of a liver specific promoter and the other one under the control of a muscle-specific promoter. Liver-specific promoter driven expression of GAA is employed to promote immune tolerance to GAA in a Gaa^{-/-} mouse model, while muscle-specific promoter driven expression of GAA provides expression of the therapeutic protein in part of the tissues targeted for therapy. However, this strategy is not entirely satisfactory in that it requires the use of multiple constructs and it does not result in body wide expression of GAA.

Modified GAA proteins have been proposed in the past to improve lysosomal storage disease treatment. In particular, application WO2004064750 and Sun et al. 2006, disclose a chimeric GAA polypeptide comprising a signal peptide operably linked to GAA as a way to enhance targeting of the protein to the secretory pathway.

However, therapies available to the patient are not entirely satisfactory and improved GAA polypeptides and GAA production is still a need in the art. In particular, a need still exists of a long term efficacy of the treatment with GAA, of high level GAA production, of improved immunological tolerance to the produced GAA polypeptide, and of improved uptake of GAA by the cells and tissues in need thereof. In addition, in WO2004064750 and Sun et al., 2006, tissue distribution of the chimeric GAA polypeptide disclosed therein is not entirely satisfactory. Therefore, a need still exists for a GAA polypeptide that would be fully therapeutic, by allowing a correction of glycogen accumulation in most if not all tissues of interest.

### SUMMARY OF THE INVENTION

The present invention relates to GAA variants that are expressed and secreted at higher levels compared to the wild type GAA protein and that elicit improved correction of the pathological accumulation of glycogen body-wide and results in the induction of immunological tolerance to GAA.

According to one aspect, the invention provides a nucleic acid molecule encoding a functional chimeric GAA polypeptide, comprising a signal peptide moiety and a functional GAA moiety. In the encoded chimeric GAA polypeptide, the endogenous (or natural) signal peptide of a GAA polypeptide is replaced with the signal peptide of another protein. The nucleic acid molecule therefore encodes a chimeric GAA polypeptide comprising a signal peptide from another protein than a GAA, operably linked to a GAA polypeptide. The encoded chimeric polypeptide is a functional GAA protein wherein the amino acid sequence corresponding to the natural signal peptide of GAA (such as that corresponding to nucleotides 1 to 81 of SEQ ID NO: 1 which is a wild-type nucleic acid encoding human GAA) is replaced by the amino acid sequence of a different protein. In a preferred embodiment, the encoded signal peptide has an amino acid sequence selected in the group consisting of SEQ ID NO:2 to 4. In a particular embodiment, the GAA moiety is a N-terminally truncated form of a parent GAA polypeptide.

In a particular embodiment, the GAA moiety has 1 to 75 consecutive amino acids deleted at its N-terminal end as compared to a parent GAA polypeptide, wherein the parent polypeptide corresponds to a precursor form of a GAA polypeptide devoid of its signal peptide. In a particular embodiment, said truncated GAA polypeptide has at least 2, in particular at least 2, in particular at least 3, in particular at least 4, in particular at least 5, in particular at least 6, in particular at least 7, in particular at least 8 consecutive amino acids deleted at its N-terminal end as compared to the parent GAA polypeptide. In another embodiment, said truncated GAA polypeptide has at most 75, in particular at most 70, in particular at most 60, in particular at most 55, in particular at most 50, in particular at most 47, in particular at most 46, in particular at most 45, in particular at most 44, in particular at most 43 consecutive amino acids deleted at its N-terminal end as compared to the parent GAA polypeptide. In a further particular embodiment, said truncated GAA polypeptide has at most 47, in particular at most 46, in particular at most 45, in particular at most 44, in particular at most 43 consecutive amino acids deleted at its N-terminal end as compared to the parent GAA polypeptide. In another particular embodiment, said truncated GAA polypeptide has 1 to 75, in particular 1 to 47, in particular 1 to 46, in particular 1 to 45, in particular 1 to 44, in particular 1 to 43 consecutive amino acids deleted at its N-terminal end as compared to the parent GAA polypeptide. In another embodiment, said truncated GAA polypeptide has 2 to 43, in particular 3 to 43, in particular 4 to 43, in particular 5 to 43, in particular 6 to 43, in particular 7 to 43, in particular 8 to 43 consecutive amino acids deleted at its N-terminal end as compared to the parent GAA polypeptide. In a more particular embodiment, said truncated GAA polypeptide has 6, 7, 8, 9, 10, 27, 28, 29, 30, 31, 40, 41, 42, 43, 44, 45, 46 or 47 consecutive amino acids deleted at its N-terminal end as compared to a parent GAA polypeptide, in particular 7, 8, 9, 28, 29, 30, 41, 42, 43 or 44, more particularly 8, 29, 42 or 43 consecutive amino acids truncated at its N-terminal end as compared to a parent GAA polypeptide. An illustrative parent GAA polypeptide is represented by the human GAA polypeptide shown in SEQ ID NO:5 or SEQ ID NO:36.

In another particular embodiment, the nucleic acid molecule of the invention is a nucleotide sequence optimized to improve the expression of and/or improve immune tolerance to the chimeric GAA *in vivo.*

In a particular embodiment, the nucleic acid molecule of the invention encodes a chimeric GAA polypeptide comprising the moieties shown in the following table 1, table 1' or table 1", in particular table 1' or table 1":

**Table 1**

| Signal peptide moiety | GAA moiety |
|---|---|
| SEQ ID NO:2 | wild-type hGAA devoid of its natural signal peptide; e.g. SEQ ID NO:5 or SEQ ID NO:36, in particular SEQ ID NO:5 |
| SEQ ID NO:3 | |
| SEQ ID NO:4 | |
| SEQ ID NO:2 | truncated hGAA deleted for 8 consecutive N-terminal amino acids; e.g. SEQ ID NO:29 |
| SEQ ID NO:3 | |
| SEQ ID NO:4 | |
| SEQ ID NO:2 | truncated hGAA deleted for 29 consecutive N-terminal amino acids; e.g. SEQ ID NO:41 |
| SEQ ID NO:3 | |
| SEQ ID NO:4 | |
| SEQ ID NO:2 | Truncated hGAA deleted for 42 consecutive N-terminal amino acids; e.g. SEQ ID NO:30 |
| SEQ ID NO:3 | |
| SEQ ID NO:4 | |
| SEQ ID NO:2 | truncated hGAA deleted for 43 consecutive N-terminal amino acids; e.g. SEQ ID NO:42 |
| SEQ ID NO:3 | |
| SEQ ID NO:4 | |
| SEQ ID NO:2 | truncated hGAA deleted for 47 consecutive N-terminal amino acids; e.g. SEQ ID NO:43 |
| SEQ ID NO:3 | |
| SEQ ID NO:4 | |

**Table 1'**

| Signal peptide moiety | GAA moiety |
|---|---|
| SEQ ID NO:2 | wild-type hGAA devoid of its natural signal peptide; e.g. SEQ ID NO:5 or SEQ ID NO:36, in particular SEQ ID NO:5 |
| SEQ ID NO:3 | |
| SEQ ID NO:4 | |
| SEQ ID NO:2 | truncated hGAA deleted for 8 consecutive N-terminal amino acids; e.g. SEQ ID NO:29 |
| SEQ ID NO:3 | |
| SEQ ID NO:4 | |
| SEQ ID NO:2 | truncated hGAA deleted for 29 consecutive N-terminal amino acids; e.g. SEQ ID NO:41 |
| SEQ ID NO:3 | |
| SEQ ID NO:4 | |
| SEQ ID NO:2 | Truncated hGAA deleted for 42 consecutive N-terminal amino acids; e.g. SEQ ID NO:30 |
| SEQ ID NO:3 | |
| SEQ ID NO:4 | |
| SEQ ID NO:2 | truncated hGAA deleted for 43 consecutive N-terminal amino acids; e.g. SEQ ID NO:42 |
| SEQ ID NO:3 | |
| SEQ ID NO:4 | |

**Table 1"**

| Signal peptide moiety | GAA moiety |
|---|---|
| SEQ ID NO:2 | wild-type hGAA devoid of its natural signal peptide; e.g. SEQ ID NO:5 or SEQ ID NO:36, in particular SEQ ID NO:5 |
| SEQ ID NO:3 | |
| SEQ ID NO:4 | |
| SEQ ID NO:2 | truncated hGAA deleted for 8 consecutive N-terminal amino acids; e.g. SEQ ID NO:29 |
| SEQ ID NO:3 | |
| SEQ ID NO:4 | |
| SEQ ID NO:2 | Truncated hGAA deleted for 42 consecutive N-terminal amino acids; e.g. SEQ ID NO:30 |
| SEQ ID NO:3 | |
| SEQ ID NO:4 | |

For example, such nucleic acid molecules may be the result of the following combinations shown in table 2, table 2' or table 2":

**Table 2**

| Signal peptide moiety coding sequence | GAA moiety coding sequence |
|---|---|
| SEQ ID NO:26 | SEQ ID NO:31 |
| SEQ ID NO:27 | |
| SEQ ID NO:28 | |
| SEQ ID NO:26 | SEQ ID NO: 13 |
| SEQ ID NO:27 | |
| SEQ ID NO:28 | |
| SEQ ID NO:26 | SEQ ID NO: 14 |
| SEQ ID NO:27 | |
| SEQ ID NO:28 | |
| SEQ ID NO:26 | SEQ ID NO:32 |
| SEQ ID NO:27 | |
| SEQ ID NO:28 | |
| SEQ ID NO:26 | SEQ ID NO:33 |
| SEQ ID NO:27 | |
| SEQ ID NO:28 | |
| SEQ ID NO:26 | SEQ ID NO:34 |
| SEQ ID NO:27 | |
| SEQ ID NO:28 | |
| SEQ ID NO:26 | SEQ ID NO:35 |
| SEQ ID NO:27 | |
| SEQ ID NO:28 | |
| SEQ ID NO:26 | SEQ ID NO:44 |
| SEQ ID NO:27 | |
| SEQ ID NO:28 | |
| SEQ ID NO:26 | SEQ ID NO:45 |
| SEQ ID NO:27 | |
| SEQ ID NO:28 | |
| SEQ ID NO:26 | SEQ ID NO:46 |
| SEQ ID NO:27 | |
| SEQ ID NO:28 | |
| SEQ ID NO:26 | SEQ ID NO:47 |
| SEQ ID NO:27 | |
| SEQ ID NO:28 | |
| SEQ ID NO:26 | SEQ ID NO:48 |
| SEQ ID NO:27 | |
| SEQ ID NO:28 | |
| SEQ ID NO:26 | SEQ ID NO:49 |
| SEQ ID NO:27 | |
| SEQ ID NO:28 | |
| SEQ ID NO:26 | SEQ ID NO:50 |
| SEQ ID NO:27 | |
| SEQ ID NO:28 | |
| SEQ ID NO:26 | SEQ ID NO:51 |
| SEQ ID NO:27 | |
| SEQ ID NO:28 | |
| SEQ ID NO:26 | SEQ ID NO:52 |
| SEQ ID NO:27 | |
| SEQ ID NO:28 | |
| SEQ ID NO:26 | SEQ ID NO:53 |
| SEQ ID NO:27 | |
| SEQ ID NO:28 | |
| SEQ ID NO:26 | SEQ ID NO:54 |
| SEQ ID NO:27 | |
| SEQ ID NO:28 | |

**Table 2'**

| Signal peptide moiety coding sequence | GAA moiety coding sequence |
|---|---|
| SEQ ID NO:26 | SEQ ID NO:31 |
| SEQ ID NO:27 | |
| SEQ ID NO:28 | |
| SEQ ID NO:26 | SEQ ID NO: 13 |
| SEQ ID NO:27 | |
| SEQ ID NO:28 | |
| SEQ ID NO:26 | SEQ ID NO:14 |
| SEQ ID NO:27 | |
| SEQ ID NO:28 | |
| SEQ ID NO:26 | SEQ ID NO:32 |
| SEQ ID NO:27 | |
| SEQ ID NO:28 | |
| SEQ ID NO:26 | SEQ ID NO:33 |
| SEQ ID NO:27 | |
| SEQ ID NO:28 | |
| SEQ ID NO:26 | SEQ ID NO:34 |
| SEQ ID NO:27 | |
| SEQ ID NO:28 | |
| SEQ ID NO:26 | SEQ ID NO:35 |
| SEQ ID NO:27 | |
| SEQ ID NO:28 | |
| SEQ ID NO:26 | SEQ ID NO:44 |
| SEQ ID NO:27 | |
| SEQ ID NO:28 | |
| SEQ ID NO:26 | SEQ ID NO:45 |
| SEQ ID NO:27 | |
| SEQ ID NO:28 | |
| SEQ ID NO:26 | SEQ ID NO:46 |
| SEQ ID NO:27 | |
| SEQ ID NO:28 | |
| SEQ ID NO:26 | SEQ ID NO:47 |
| SEQ ID NO:27 | |
| SEQ ID NO:28 | |
| SEQ ID NO:26 | SEQ ID NO:48 |
| SEQ ID NO:27 | |
| SEQ ID NO:28 | |
| SEQ ID NO:26 | SEQ ID NO:49 |
| SEQ ID NO:27 | |
| SEQ ID NO:28 | |
| SEQ ID NO:26 | SEQ ID NO:50 |
| SEQ ID NO:27 | |
| SEQ ID NO:28 | |
| SEQ ID NO:26 | SEQ ID NO:51 |
| SEQ ID NO:27 | |
| SEQ ID NO:28 | |

**Table 2"**

| Signal peptide moiety coding sequence | GAA moiety coding sequence |
|---|---|
| SEQ ID NO:26 | SEQ ID NO:31 |
| SEQ ID NO:27 | |
| SEQ ID NO:28 | |
| SEQ ID NO:26 | SEQ ID NO:13 |
| SEQ ID NO:27 | |
| SEQ ID NO:28 | |
| SEQ ID NO:26 | SEQ ID NO:14 |
| SEQ ID NO:27 | |
| SEQ ID NO:28 | |
| SEQ ID NO:26 | SEQ ID NO:32 |
| SEQ ID NO:27 | |
| SEQ ID NO:28 | |
| SEQ ID NO:26 | SEQ ID NO:33 |
| SEQ ID NO:27 | |
| SEQ ID NO:28 | |
| SEQ ID NO:26 | SEQ ID NO:34 |
| SEQ ID NO:27 | |
| SEQ ID NO:28 | |
| SEQ ID NO:26 | SEQ ID NO:35 |
| SEQ ID NO:27 | |
| SEQ ID NO:28 | |

In yet another aspect, the invention relates to a nucleic acid construct, comprising the nucleic acid molecule of the invention operably linked to one or more regulatory sequences such as a promoter, an intron, a polyadenylation signal and/or an enhancer (for example a cis-regulatory module, or CRM). In a particular embodiment, the promoter is a liver-specific promoter preferably selected in the group consisting of the alpha-1 antitrypsin promoter (hAAT), the transthyretin promoter, the albumin promoter and the thyroxine-binding globulin (TBG) promoter. In another particular embodiment, the promoter is a muscle-specific promoter, such as the Spc5-12, MCK and desmin promoters. In another embodiment, the promoter is an ubiquitous promoter such as the CMV, CAG and PGK promoters. The nucleic acid construct may further optionally comprises an intron, in particular an intron selected in the group consisting of a human beta globin b2 (or HBB2) intron, a FIX intron, a chicken beta-globin intron and a SV40 intron, wherein said intron is optionally a modified intron such as a modified HBB2 intron of SEQ ID NO:7, a modified FIX intron of SEQ ID NO:9, or a modified chicken beta-globin intron of SEQ ID NO: 11.

In another particular embodiment, the nucleic acid construct comprises, preferably in this order: an enhancer; an intron; a promoter, in particular a liver-specific promoter; the nucleic acid sequence encoding the chimeric GAA polypeptide; and a polyadenylation signal, the construct comprising preferably, in this order: an ApoE control region; a HBB2 intron, in particular a modified HBB2 intron; a hAAT promoter; the nucleic acid sequence encoding the chimeric GAA polypeptide; and a bovine growth hormone polyadenylation signal. In specific embodiment, the nucleic acid construct comprises a nucleotide sequence selected in the group consisting of the combinations of sequences shown in table 2, table 2' or table 2", in particular in table 2' or 2", more particularly the nucleotide sequence of SEQ ID NO:17 (corresponding to the fusion of SEQ ID NO:26 and SEQ ID NO:32), 18 (corresponding to the fusion of SEQ ID NO:27 and SEQ ID NO:32) or 19 (corresponding to the fusion of SEQ ID NO:28 and SEQ ID NO:32).

In another aspect, the invention relates to a cell transformed with the nucleic acid molecule or the nucleic acid construct of the invention. In a particular embodiment, the cell is a liver cell or a muscle cell.

According to another aspect, the invention relates to a chimeric GAA polypeptide, comprising a signal peptide moiety and a functional GAA moiety. The signal peptide moiety is selected in the group consisting of SEQ ID NO:2 to 4, preferably SEQ ID NO:2. Furthermore, the GAA moiety may be a truncated form of a parent GAA polypeptide, such as a GAA moiety having 1 to 75 consecutive amino acids truncated at its N-terminal end as compared to a parent GAA polypeptide, in particular 6, 7, 8, 9, 10, 20, 41, 42, 43 or 44 consecutive amino acids truncated at its N-terminal end as compared to a parent GAA polypeptide, such as 8 or 42 consecutive amino acids truncated at its N-terminal end as compared to a parent GAA polypeptide, wherein the GAA moiety is in particular a truncated form of the human GAA protein of SEQ ID NO:5 or SEQ ID NO:36, in particular of SEQ ID NO:5. In a particular embodiment, the GAA moiety has 8 consecutive amino acids truncated at its N-terminal end as compared to a parent GAA polypeptide (more particularly the parent GAA polypeptide of SEQ ID NO:5 or SEQ ID NO:36, in particular of SEQ ID NO:5). In a particular embodiment of the invention, the chimeric GAA polypeptide of the invention is selected in the group consisting of the combinations of amino acid sequences shown in table 1, table 1' or table 1", in particular in table 1' or table 1". Further particular embodiments of the chimeric GAA polypeptide comprising a truncated for of a parent GAA polypeptide are disclosed in the following detailed description.

In another aspect, the invention relates to a pharmaceutical composition, comprising, in a pharmaceutically acceptable carrier, the nucleic acid sequence, the nucleic acid construct, the cell or the chimeric polypeptide disclosed herein.

Another aspect of the invention relates to the nucleic acid sequence, the nucleic acid construct, the cell, or the chimeric polypeptide of the invention, for use as a medicament.

In yet another aspect, the invention relates to the nucleic acid sequence, the nucleic acid construct, the cell, or the chimeric polypeptide of the invention, for use in a method for treating a glycogen storage disease. In a particular embodiment, the glycogen storage disease is GSDI, GSDII, GSDIII, GSDIV, GSDV, GSDVI, GSDVII, GSDVIII or lethal congenital glycogen storage disease of the heart. In a more particular embodiment, the glycogen storage disease is selected in the group consisting of GSDI, GSDII and GSDIII, more particularly in the group consisting of GSDII and GSDIII. In an even more particular embodiment, the glycogen storage disease is GSDII.

### LEGENDS TO THE FIGURES

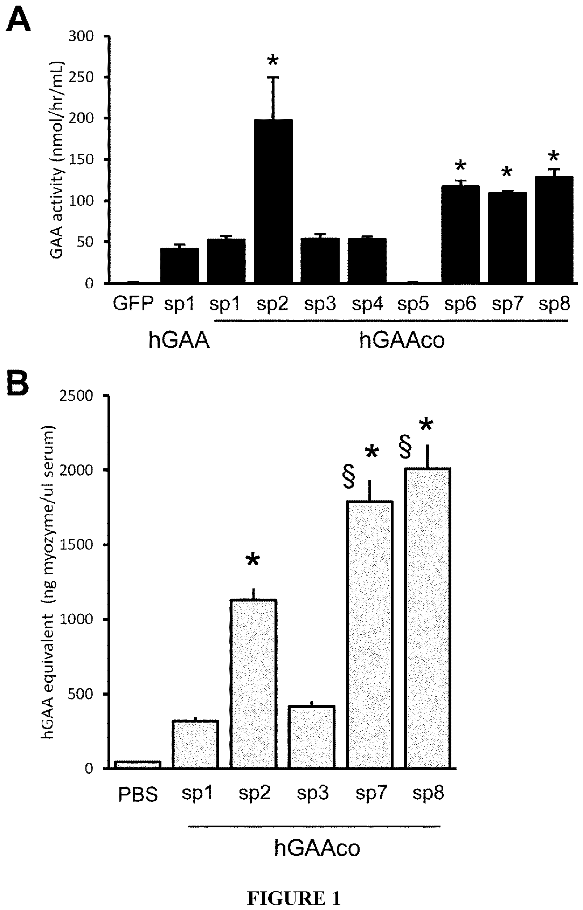

**Figure 1****.** Signal peptides enhance secretion of hGAA to a variable extent in vitro and in vivo. **Panel A**. Human hepatoma cells (Huh7) were transfected by Lipofectamine^{™} with a control plasmid (GFP), a plasmid expressing wild-type hGAA under the transcriptional control of a liver specific promoter (noted as sp1), or plasmids expressing sequence optimized hGAA (hGAAco) fused with signal peptides 1-8 (sp2 (sp1-8) of synthetic origin or derived from other highly-secreted proteins. 48 hours after transfection the activity of hGAA in the culture media was measured by a fluorogenic enzymatic assay and GAA activity evaluated against a standard curve of 4-methylumbelliferone. The histogram plot shows the average ± SE of the levels of secreted hGAA deriving from three different experiments. Statistical analysis has been performed by ANOVA (* = p<0.05 vs mock transfected cells). **Panel B.** The histogram plot shows the average ± SE of the activity of hGAA in serum of 3-month-old C57B16J mice (n=5 mice/group) 1 month after the injection of PBS (PBS) or 1E12 vg/kg of AAV8 vectors expressing sequence optimized hGAA (hGAAco) under the transcriptional control of human alpha-1-antytripsin promoter and fused with signal peptides 1 to 3 and 7-8 (sp1-3, 7-8). The activity of hGAA in serum has been quantified by a fluorogenic enzymatic assay and GAA activity evaluated against a standard curve of recombinant hGAA protein. Statistical analysis has been performed by ANOVA (* = p<0.05 vs PBS injected, § = p<0.05 vs sp2).

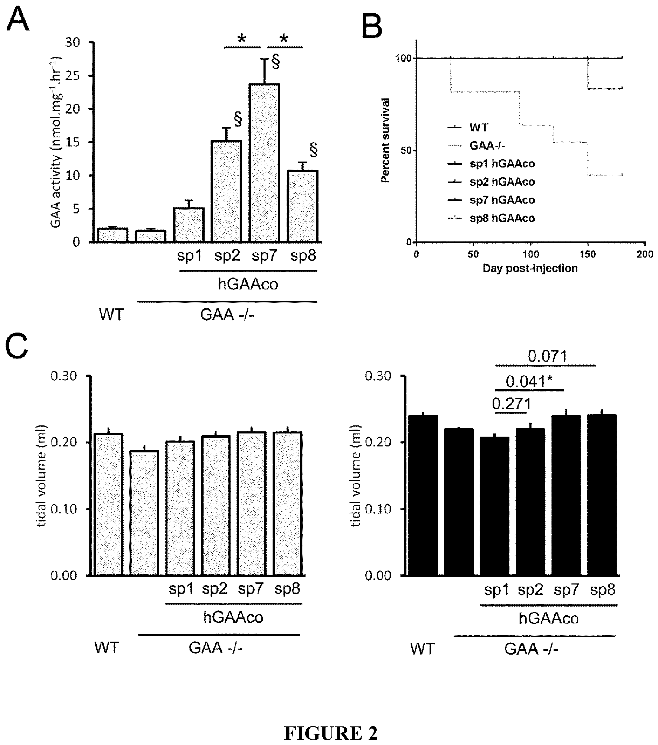

**Figure 2****.** sp7 signal peptide increases levels of circulating hGAA and rescue the respiratory impairment in a Pompe disease mouse model. 4 months-old wild type (WT) and GAA^{-/-} mice (n=6-9 mice/group) were intravenously injected with PBS or 2E12 vg/kg of AAV8 vectors expressing sequence optimized hGAA (hGAAco) under the transcriptional control of human alpha-1-antytripsin promoter and fused with signal peptides 1, 2, 7 and 8 (sp1, 2, 7, 8). **Panel A.** The histogram plot shows the hGAA activity measured by fluorogenic assay in blood three months after vectors injection. Statistical analysis has been performed by ANOVA (* = p<0.05 as indicated, § = p<0.05 vs sp1 treated mice). **Panel B.** Kaplan-Mayer survival curve measured on mice treated as described above and followed for 6 months. Statistical analysis has been performed by log-rank test (* = p<0.05). **Panel C.** Respiratory function assessment. Histograms show the tidal volume, in milliliters (ml) measured three (gray bars) and six (black bars) months after the treatment with indicated vectors. Statistical analysis has been performed by ANOVA, in the histogram are reported the p-values obtained vs sp1 treated GAA -/- animals (^{∗} = p<0.05).

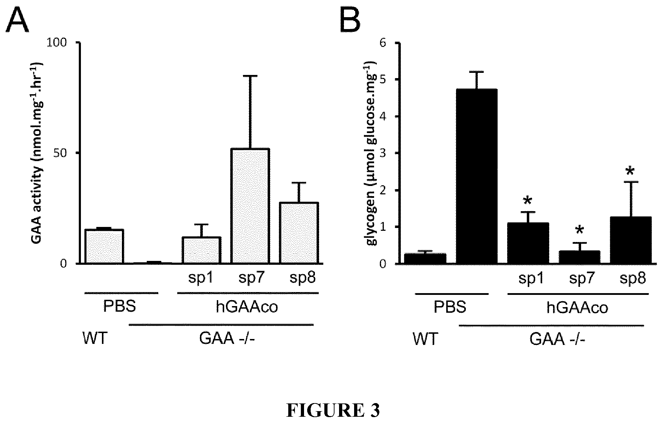

**Figure 3****.** Biochemical correction of glycogen content in quadriceps. 4 months-old GAA^{-/-} mice were intravenously injected with PBS or 2E12 vg/kg of AAV8 vectors expressing sequence optimized hGAA (hGAAco) under the transcriptional control of human alpha-1-antytripsin promoter and fused with signal peptides 1, 7 and 8 (sp1, 7, 8). **Panel A.** hGAA activity measured by fluorogenic assay in quadriceps. **Panel B.** In the histogram is shown the glycogen content expressed as glucose released after enzymatic digestion of glycogen, measured in the quadriceps. Statistical analysis has been performed by ANOVA (*=p<0.05 vs PBS injected GAA -/- mice).

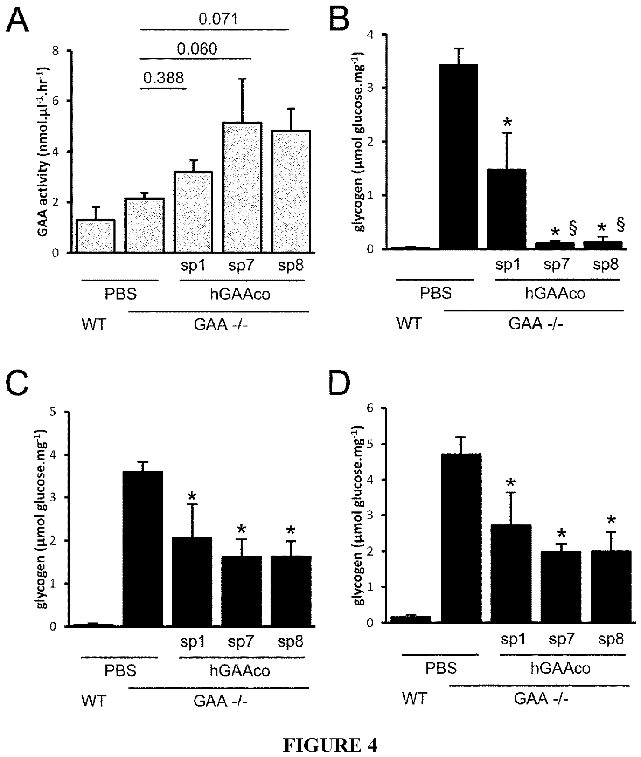

**Figure 4****.** Biochemical correction of glycogen content in heart, diaphragm and quadriceps. 4 months-old wild type (WT) and GAA^{-/-} mice (n=4-5 mice/group) were intravenously injected with PBS 6E11 vg/kg of AAV8 vectors expressing sequence optimized hGAA (hGAAco) under the transcriptional control of human alpha-1-antytripsin promoter and fused with signal peptides 1, 7 and 8 (sp1, 7, 8). **Panel A.** The histogram plot shows the hGAA activity measured by fluorogenic assay in blood three months after vector injection. Statistical analysis has been performed by ANOVA, in the histogram are reported the p-values obtained vs PBS treated GAA -/- animals (* = p<0.05). **Panel B-D.** The histogram plots show the glycogen content expressed as glucose released after enzymatic digestion of glycogen, measured in the heart (**panel B**), diaphragm (**panel C**) and quadriceps (**panel D**). Statistical analysis has been performed by ANOVA (* = p<0.05 vs PBS injected GAA -/- mice, § = p<0,05 vs. sp1-treated mice).

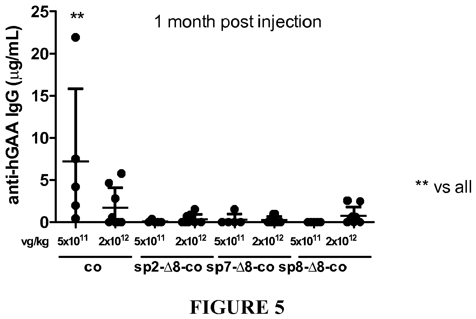

**Figure 5****.** Highly secreted hGAA reduces humoral responses directed against the transgene in a Pompe disease mouse model. 4 months-old GAA-/- mice were intravenously injected with PBS or with two different doses (5E11 or 2E12 vg/kg) of AAV8 vectors comprising an optimized sequence under the transcriptional control of human alpha-1-antytripsin promoter, encoding Δ8 hGAA, fused to signal peptide 1 (co), signal peptide 2 (sp2-Δ8-co), signal peptide 7 (sp7-Δ8-co) or signal peptide 8 (sp8-Δ8-co). 1 month after the injections, sera were analyzed for the presence of anti-hGAA antibodies by ELISA. The quantification has been performed using purified mouse IgG as standard. Statistical analysis has been performed by ANOVA with Dunnett's post-hoc test (* = p<0.01).

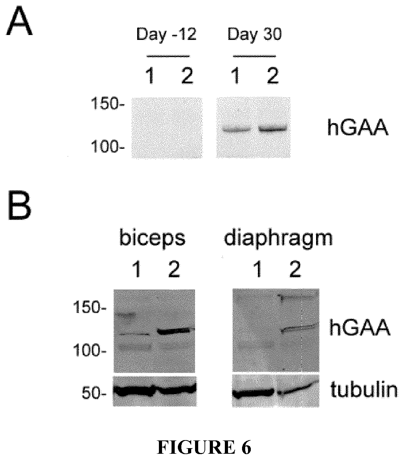

**Figure 6****.** AAV8-hAAT-sp7-Δ8-hGAAco1 injection leads to efficacious secretion of hGAA in the blood and uptake in muscle in NHP. Two *Macaca Fascicularis* monkeys were injected at day 0 with 2E12 vg/kg of AAV8-hAAT-sp7-Δ8-hGAAco1.**Panel A** hGAA western blot performed on serum from the two monkeys obtained twelve days before and 30 days after vector administration. On the left are indicated the positions of the bands of the molecular weight marker (st) running in parallel with the samples. **Panel B** Three months after vector injection the monkeys were sacrificed and tissues harvested for biochemical evaluation of hGAA uptake. A hGAA Western blot was performed on tissue extracts obtained from biceps and diaphragm. An anti-tubulin antibody was used as loading control. On the left are indicated the positions of the bands of the molecular weight marker running in parallel with the samples.

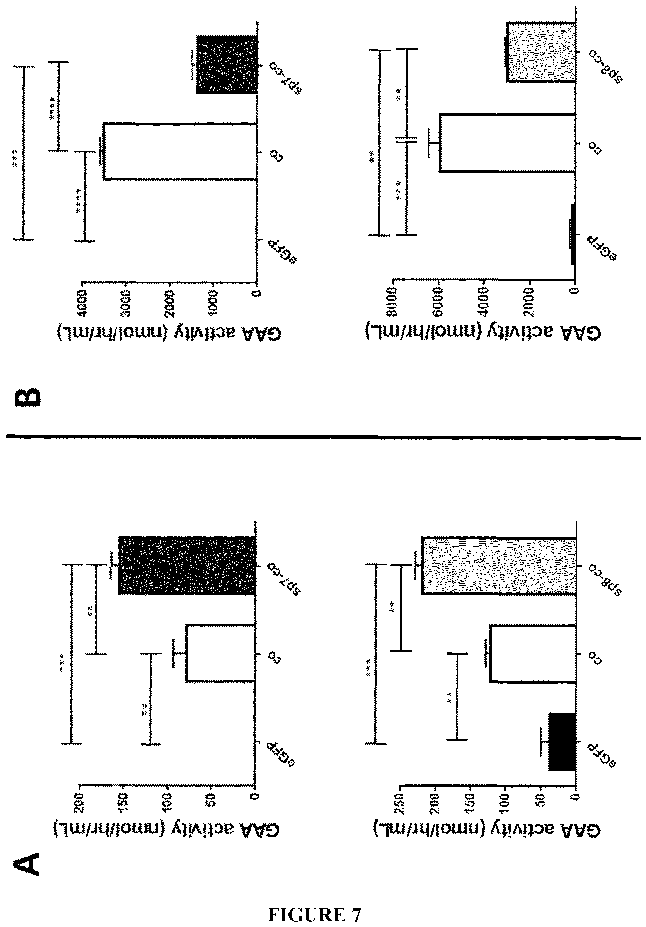

**Figure 7****.** Increased GAA activity in media of cells transfected with plasmids encoding GAA variants combined with heterologous sp7 or sp8 signal peptide. GAA activity measured in the media (panels A) and lysates (panels B) of HuH7 cells 48 hours following transfection of plasmids comprising optimized sequences encoding native GAA combined to the native GAA sp1 signal peptide (co) or encoding engineered GAA including native GAA combined to the heterologous sp7 or sp8 signal peptide (sp7-co or sp8-co). A plasmid encoding for eGFP was used as negative control. Statistical analysis was performed by One-way ANOVA with Tukey post-hoc. Data are average±SD of two independent experiments. ^{∗}p<0.05, ^{∗∗}p<0.01, ^{∗∗∗}p<0.001, ^{∗∗∗∗}p<0.0001.

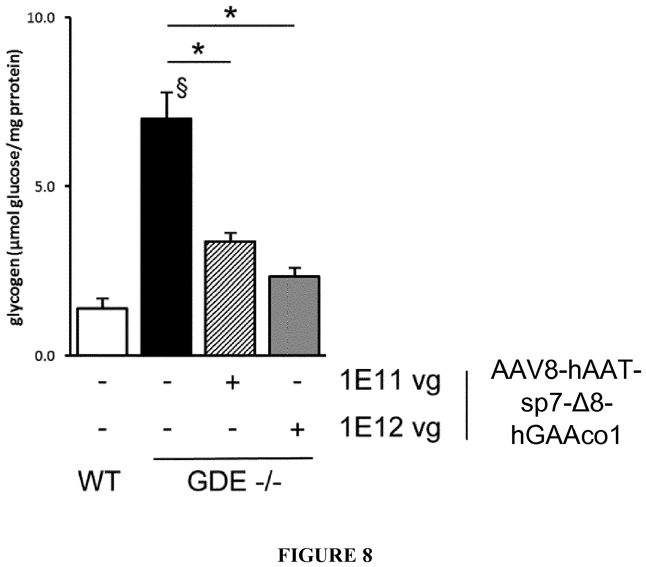

**Figure 8****.** Biochemical correction of glycogen content in the liver of GDE -/- animals injected with hGAA expressing vector. 3 months-old wild-type (WT) or GDE -/-mice were intravenously injected with PBS or AAV8 vectors expressing codon optimized hGAA under the transcriptional control of human alpha-1-antytripsin promoter and fused with signal peptide 7 (AAV8-hAAT-sp7--Δ8-hGAAco1) at the dose of 1E11 or 1E12 vg/mouse. The histogram plot shows the glycogen content expressed as glucose released after enzymatic digestion of glycogen, measured in the liver. Statistical analysis was performed by ANOVA (*=p<0.05 vs PBS injected GDE -/- mice, §=p<0.05 vs PBS injected WT animals).

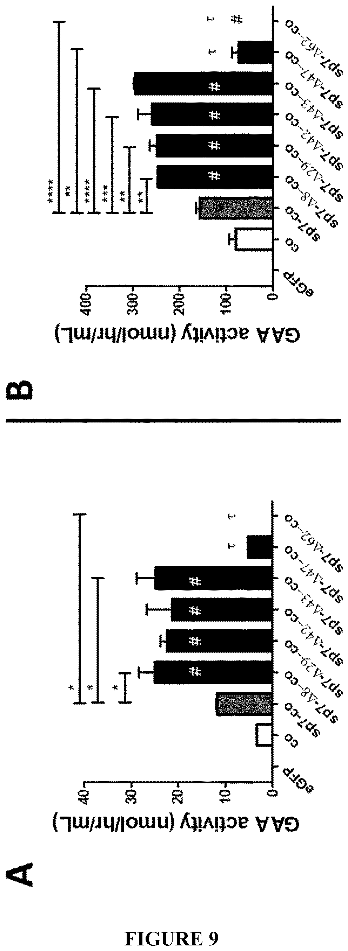

**Figure 9****.** GAA activity in media of cells transfected with plasmids encoding different GAA variants. GAA activity was measured in the media of HuH7 cells 24 (panel A) and 48 hours (panel B) following transfection of plasmids comprising optimized sequences encoding native GAA combined to the native GAA sp1 signal peptide (co) or encoding engineered GAA including native GAA combined to the heterologous sp7 signal peptide (sp7-co). The effect of different deletions in the GAA coding sequence after the sp7 signal peptide was evaluated (sp7-Δ8-co, sp7-Δ29-co, sp7-Δ42-co, sp7-Δ43-co, sp7-Δ47-co, sp7-Δ62-co). A plasmid encoding for eGFP was used as negative control. Statistical analysis was performed by One-way ANOVA with Tukey post-hoc. Hash marks (#) in the bars show statistically significant differences vs. co; tau symbols (r) show statistically significant differences vs. sp7-Δ8-co, sp7-Δ29-co, sp7-Δ42-co, sp7-Δ43-co. Data are average±SD of two independent experiments. *p<0.05, **p<0.01, ***p<0.001, ****p<0.0001 except where different symbols are used.

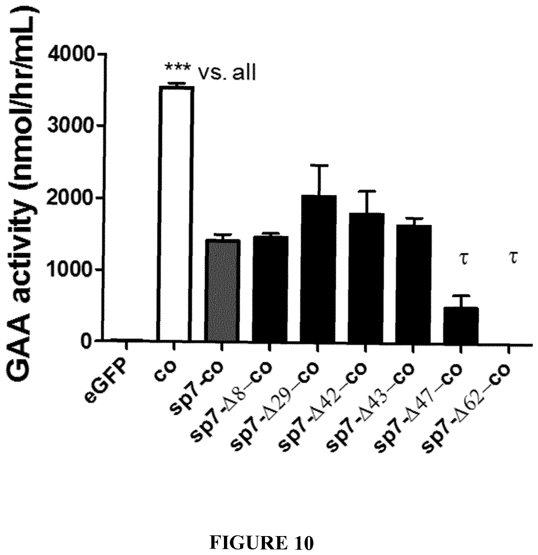

**Figure 10****.** Intracellular GAA activity of different GAA variants. GAA activity was measured in the lysates of HuH7 cells 48 hours following transfection of plasmids comprising optimized sequences encoding native GAA combined to the native GAA sp 1 signal peptide (co) or encoding engineered GAA including native GAA combined to the heterologous sp7 signal peptide (sp7-co). The effect of different deletions in the GAA coding sequence after the signal peptide was evaluated (sp7-Δ8-co, sp7-Δ29-co, sp7-Δ42-co, sp7-Δ43-co, sp7-Δ47-co, sp7-Δ62-co). A plasmid encoding for eGFP was used as negative control. Statistical analysis was performed by One-way ANOVA with Tukey post-hoc. Tau symbols (τ) show statistically significant differences vs. sp7-co, sp7-Δ8-co, sp7-Δ29-co, sp7-Δ42-co, sp7-Δ43-co. Data are average±SD of two independent experiments. ^{∗}p<0.05, ^{∗∗}p<0.01, ^{∗∗∗}p<0.001, ^{∗∗∗∗}p<0.0001 except where different symbols are used.

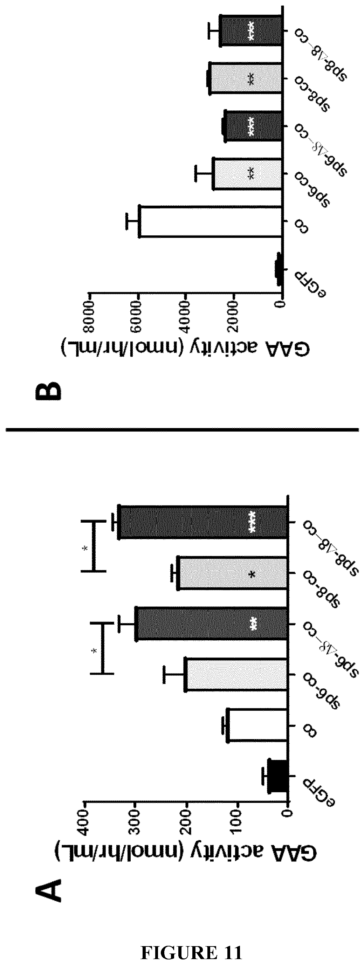

**Figure 11****.** Increased GAA activity in cell media using the Δ8 deletion combined with the sp6 or sp8 signal peptides. GAA activity was measured in the media (panel A) and lysates (panel B) of HuH7 cells 48 hours following transfection of plasmids comprising optimized sequences encoding native GAA combined to the native GAA sp 1 signal peptide (co) or encoding engineered GAA including native GAA combined to the heterologous sp6 or sp8 signal peptide (sp6-co or sp8-co). The effect of the deletion of 8 amino-acids in the GAA coding sequence after the signal peptide is evaluated (sp6-Δ8-co, sp8-Δ8-co). A plasmid encoding eGFP was used as negative control. Statistical analysis was performed by One-way ANOVA with Tukey post-hoc. Asterics in the bars shows statistically significant differences vs. co. Data are average±SD of two independent experiments. ^{∗}p<0.05, ^{∗∗}p<0.01, ^{∗∗∗}p<0.001, ^{∗∗∗∗}p<0.0001 except where different symbols are used.

### DETAILED DESCRIPTION OF THE INVENTION

The present invention relates to a nucleic acid molecule encoding a chimeric GAA polypeptide. This chimeric GAA polypeptide comprises a signal peptide moiety and a functional GAA moiety, wherein the signal peptide moiety is selected in the group consisting of SEQ ID NO:2 to 4. The inventors have surprisingly shown that fusion of one of these signal peptides to a GAA protein greatly improves GAA secretion while reducing its immunogenicity.

Lysosomal acid α-glucosidase or "GAA" (E.C. 3.2. 1.20) (1,4-α-D-glucan glucohydrolase), is an exo-1,4-α-D-glucosidase that hydrolyses both α-1,4 and α-1,6 linkages of oligosaccharides to liberate glucose. A deficiency in GAA results in glycogen storage disease type II (GSDII), also referred to as Pompe disease (although this term formally refers to the infantile onset form of the disease). It catalyzes the complete degradation of glycogen with slowing at branching points. The 28 kb human acid α-glucosidase gene on chromosome 17 encodes a 3.6 kb mRNA which produces a 952 amino acid polypeptide (Hoefsloot et al., (1988) EMBO J. 7: 1697; Martiniuk et al., (1990) DNA and Cell Biology 9: 85). The enzyme receives co-translational N-linked glycosylation in the endoplasmic reticulum. It is synthesized as a 110-kDa precursor form, which matures by extensive glycosylation modification, phosphorylation and by proteolytic processing through an approximately 90-kDa endosomal intermediate into the final lysosomal 76 and 67 kDa forms (Hoefsloot, (1988) EMBO J. 7: 1697; Hoefsloot et al., (1990) Biochem. J. 272: 485; Wisselaar et al., (1993) J. Biol. Chem. 268: 2223; Hermans etal., (1993) Biochem. J. 289: 681).

In patients with GSD II, a deficiency of acid α-glucosidase causes massive accumulation of glycogen in lysosomes, disrupting cellular function (Hirschhorn, R. and Reuser, A. J. (2001), in The Metabolic and Molecular Basis for Inherited Disease, (eds, Scriver, C. R. et al.) pages 3389-3419 (McGraw-Hill, New York). In the most common infantile form, patients exhibit progressive muscle degeneration and cardiomyopathy and die before two years of age. Severe debilitation is present in the juvenile and adult onset forms.

Furthermore, patients having other GSDs may benefit from the administration of an optimized form of GAA. For example, it has been shown (Sun et al. (2013) Mol Genet Metab 108(2): 145; WO2010/005565) that administration of GAA reduces glycogen in primary myoblasts from glycogen storage disease type III (GSD III) patients.

The term "GAA" or "GAA polypeptide", as used herein, encompasses mature (~76 or ~67 kDa) and precursor (e.g., ~110 kDa) GAA, in particular the precursor form, as well as modified or mutated by insertion(s), deletion (s) and/or substitution(s)) GAA proteins or fragments thereof that are functional derivatives of GAA, i.e. that retain biological function of GAA (i.e., have at least one biological activity of the native GAA protein, e. g., can hydrolyze glycogen, as defined above) and GAA variants (e.g., GAA II as described by Kunita et al., (1997) Biochemica et Biophysica Acta 1362: 269; GAA polymorphisms and SNPs are described by Hirschhorn, R. and Reuser, A. J. (2001) In The Metabolic and Molecular Basis for Inherited Disease (Scriver, C. R., Beaudet, A. L., Sly, W. S. & Valle, D. Eds. ), pp. 3389- 3419. McGraw-Hill, New York, see pages 3403-3405). Any GAA coding sequence known in the art may be used, for example, see SEQ ID NO:1; GenBank Accession number NM_00152 and Hoefsloot et al., (1988) EMBO J. 7: 1697 and Van Hove et al., (1996) Proc. Natl. Acad. Sci. USA 93: 65 (human), GenBank Accession number NM_008064 (mouse), and Kunita et al., (1997) Biochemica et Biophysica Acta 1362: 269 (quail).

In the context of the present invention, a "precursor form of GAA" is a form of the GAA polypeptide that comprises its natural signal peptide. For example, the sequence of SEQ ID NO: 12 and SEQ ID NO:37 are the precursor forms of human GAA (hGAA) variants. Within SEQ ID NO: 12 and SEQ ID NO:37, amino acid residues 1-27 correspond to the signal peptide of the hGAA polypeptide.

In the context of the present invention, a truncated GAA polypeptide of the invention is derived from a parent GAA polypeptide. According to the present invention a "parent GAA polypeptide" may be a functional, precursor GAA sequence as defined above, but devoid of its signal peptide. For example, with reference to wild-type human GAA polypeptide, a complete wild-type GAA polypeptide (i.e. the precursor form of GAA) is represented in SEQ ID NO: 12 or SEQ ID NO:37 and has a signal peptide (corresponding to amino acids 1-27 of SEQ ID NO: 12 or SEQ ID NO:37), whereas the parent GAA polypeptide serving as basis for the truncated GAA forms of these wild-type human GAA polypeptides are represented in SEQ ID NO:5 and SEQ ID NO:36 and have no signal peptide. In this example, the latter, corresponding to amino acids 28-952 of SEQ ID NO: 12 and to amino acids 28-952 of SEQ ID NO37, is referred to as a parent GAA polypeptide.

The coding sequence of the GAA polypeptide can be derived from any source, including avian and mammalian species. The term "avian" as used herein includes, but is not limited to, chickens, ducks, geese, quail, turkeys and pheasants. The term "mammal" as used herein includes, but is not limited to, humans, simians and other non-human primates, bovines, ovines, caprines, equines, felines, canines, lagomorphs, etc. In embodiments of the invention, the nucleic acids of the invention encode a human, mouse or quail, in particular a human, GAA polypeptide. In a further particular embodiment, the GAA polypeptide encoded by the nucleic acid molecule of the invention comprises the amino acid sequence shown in SEQ ID NO:5 or in SEQ ID NO:36, which corresponds to hGAA without its signal peptide (of note, the natural signal peptide of hGAA corresponds to amino acid 1-27 in SEQ ID NO: 12 or in SEQ ID NO:37, which corresponds to hGAA including its natural signal peptide).

In another embodiment of the invention, the nucleic acid molecule of the invention has at least 75 percent (such as at least 77%), at least 80 percent or at least 82 percent (such as at least 83%) identify to nucleotides 82-2859 of the sequence shown in SEQ ID NO:1, which is the sequence coding the wild-type hGAA of SEQ ID NO:37 (nucleotides 1-81 of SEQ ID NO: 1 being the part encoding for the natural signal peptide of hGAA).

The GAA moiety of the nucleic acid molecule of the invention preferably has at least 85 percent, more preferably at least 90 percent, and even more preferably at least 92 percent identity, in particular at least 95 percent identity, for example at least 98, 99 or 100 percent identity to the nucleotide sequence of SEQ ID NO: 13 or 14, which are sequences optimized for transgene expression *in vivo.*

In addition, the signal peptide moiety of the chimeric GAA protein encoded by the nucleic acid molecule of the invention may comprise from 1 to 5, in particular from 1 to 4, in particular from 1 to 3, more particularly from 1 to 2, in particular 1 amino acid deletion(s), insertion(s) or substitution(s) as compared to the sequences shown in SEQ ID NO:2 to 4, as long as the resulting sequence corresponds to a functional signal peptide, i.e. a signal peptide to that allows secretion of a GAA protein. In a particular embodiment, the signal peptide moiety sequence consists of a sequence selected in the group consisting of SEQ ID NO:2 to 4.

The term "identical" and declinations thereof refers to the sequence identity between two nucleic acid molecules. When a position in both of the two compared sequences is occupied by the same base e.g., if a position in each of two DNA molecules is occupied by adenine, then the molecules are identical at that position. The percent of identity between two sequences is a function of the number of matching positions shared by the two sequences divided by the number of positions compared X 100. For example, if 6 of 10 of the positions in two sequences are matched then the two sequences are 60% identical. Generally, a comparison is made when two sequences are aligned to give maximum identity. Various bioinformatic tools known to the one skilled in the art might be used to align nucleic acid sequences such as BLAST or FASTA.

In a particular embodiment, the GAA moiety of the nucleic acid molecule of the invention comprises the sequence shown in SEQ ID NO: 13 or SEQ ID NO: 14.

The nucleic acid molecule of the invention encodes a functional GAA polypeptide, i.e. it encodes for a human GAA polypeptide that, when expressed, has the functionality of wild-type GAA protein. As defined above, the functionality of wild-type GAA is to hydrolyse both α-1,4 and α-1,6 linkages of oligosaccharides and polysaccharides, more particularly of glycogen, to liberate glucose. The functional GAA polypeptide encoded by the nucleic acid of the invention may have a hydrolysing activity on glycogen of at least 50 %, 60 %, 70 %, 80 %, 90 %, 95 %, 99 %, or at least 100 % as compared to the wild-type GAA polypeptide encoded by the nucleic acid sequence of SEQ ID NO:1, SEQ ID NO: 13 or SEQ ID NO:14 (i.e. the GAA polypeptide having the amino acid sequence of SEQ ID NO:5). The activity of the GAA protein encoded by the nucleic acid of the invention may even be of more than 100 %, such as of more than 110 %, 120 %, 130 %, 140 %, or even more than 150 % of the activity of the wild-type GAA polypeptide encoded by the nucleic acid sequence of SEQ ID SEQ ID NO: 1, NO: 13 or SEQ ID NO:14 (i.e. the GAA polypeptide having the amino acid sequence of SEQ ID NO:5).

A skilled person is readily able to determine whether a nucleic acid according to the invention expresses a functional GAA protein. Suitable methods would be apparent to those skilled in the art. For example, one suitable in vitro method involves inserting the nucleic acid into a vector, such as a plasmid or viral vector, transfecting or transducing host cells, such as 293T or HeLa cells, or other cells such as Huh7, with the vector, and assaying for GAA activity. Alternatively, a suitable *in vivo* method involves transducing a vector containing the nucleic acid into a mouse model of Pompe disease or another glycogen storage disorder and assaying for functional GAA in the plasma of the mouse and presence of GAA in tissues. Suitable methods are described in more details in the experimental part below.

The inventors have found that the above described nucleic acid molecule causes surprisingly high levels of expression of functional GAA protein both *in vitro* and *in vivo* compared to the wild-type GAA cDNA. Furthermore, as also shown by the inventors, the chimeric GAA polypeptide produced from liver and muscle cells expressing the nucleic acid molecule of the invention induces no humoral immune response against the transgene. This means that this nucleic acid molecule may be used to produce high levels of GAA polypeptide, and provides therapeutic benefits such as avoiding to resort to immunosuppressive treatments, allowing low dose immunosuppressive treatment, and allowing repeated administration of the nucleic acid molecule of the invention to a subject in need thereof. Therefore, the nucleic acid molecule of the invention is of special interest in contexts where GAA expression and/or activity is deficient or where high levels of expression of GAA can ameliorate a disease, such as for a glycogen storage disease. In a particular, the glycogen storage disease may be GSDI (von Gierke's disease), GSDII (Pompe disease), GSDIII (Cori disease), GSDIV, GSDV, GSDVI, GSDVII, GSDVIII or lethal congenital glycogen storage disease of the heart. More particularly, the glycogen storage disease is selected in the group consisting of GSDI, GSDII and GSDIII, even more particularly in the group consisting of GSDII and GSDIII. In an even more particular embodiment, the glycogen storage disease is GSDII. In particular, the nucleic acid molecules of the invention may be useful in gene therapy to treat GAA-deficient conditions, or other conditions associated by accumulation of glycogen such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII. In an even more particular embodiment, the nucleic acid molecules of the invention may be useful in gene therapy to treat GSDII.

The sequence of the nucleic acid molecule of the invention, encoding a functional GAA, is optimized for expression of the GAA polypeptide *in vivo.* Sequence optimization may include a number of changes in a nucleic acid sequence, including codon optimization, increase of GC content, decrease of the number of CpG islands, decrease of the number of alternative open reading frames (ARFs) and decrease of the number of splice donor and splice acceptor sites. Because of the degeneracy of the genetic code, different nucleic acid molecules may encode the same protein. It is also well known that the genetic codes of different organisms are often biased towards using one of the several codons that encode the same amino acid over the others. Through codon optimization, changes are introduced in a nucleotide sequence that take advantage of the codon bias existing in a given cellular context so that the resulting codon optimized nucleotide sequence is more likely to be expressed in such given cellular context at a relatively high level compared to the non-codon optimised sequence. In a preferred embodiment of the invention, such sequence optimized nucleotide sequence encoding a truncated GAA is codon-optimized to improve its expression in human cells compared to non-codon optimized nucleotide sequences coding for the same truncated GAA protein, for example by taking advantage of the human specific codon usage bias.

Table 3 provides a description of relevant parameters with respect to sequence optimization conducted by the inventors:

**Table 3. Description of the optimized sequences. Table illustrating the characteristics of the two hGAA optimized sequences compared to the wild-type one. a) codon adaptation index and b) GC content calculated using a rare codon analysis tool (http://www.genscript.com). c) and d) are respectively the alternative open reading frames calculated on the 5' to 3' (aORF 5'→3')and 3' to 5' (aORF 3'→5')strands. e) and f) are respectively the acceptor (SA) and donor (SD) splicing sites calculated using a splicing site online prediction tool (http://www.fruitfly.org/seq_tools/splice.html). g) and h) are respectively the percentual identity calculated versus wild-type (wt) and optimized co1 sequence. i) CpG islands calculated using MethDB online tool (http://www.methdb.de/links.html). CpG islands are sequences longer than 100 bp, with GC content>60% and an observed/expected ratio>0.6.**

| sequence | WT | co1 | co2 |
|---|---|---|---|
| CAI^{a} | 0.84 | 0.94 | 0.77 |
| GC content^{b} | 64.7 | 61.9 | 54.4 |
| aORF 5'→3'^{c} | 2 | 3 | 0 |
| aORF 3'→5'^{d} | 5 | 4 | 0 |
| SA^{e} | 3 | 0 | 1 |
| SD^{f} | 3 | 0 | 0 |
| % identity vs wt^{g} | | 83.1 | 77.7 |
| % identity vs co1^{h} | | | 80.8 |
| CpG islands¹ | 4 | 5 | 1 |

In a particular embodiment, the optimized GAA coding sequence is codon optimized, and/or has an increased GC content and/or has a decreased number of alternative open reading frames, and/or has a decreased number of splice donor and/or splice acceptor sites, as compared to nucleotides 82-2859 of the wild-type hGAA coding sequence of SEQ ID NO:1. For example, nucleic acid sequence of the invention results in an at least 2, 3, 4, 5 or 10 % increase of GC content in the GAA sequence as compared to the sequence of the wild-type GAA sequence. In a particular embodiment, the nucleic acid sequence of the invention results in a 2, 3, 4 or, more particularly, 5% or 10% (particularly 5%) increase of GC content in the GAA sequence as compared to the sequence of the wild-type GAA nucleotide sequence. In a particular embodiment, the nucleic acid sequence of the invention encoding a functional GAA polypeptide is "substantially identical", that is, about 70% identical, more preferably about 80% identical, even more preferably about 90% identical, even more preferably about 95% identical, even more preferably about 97%, 98% or even 99% identical to nucleotides 82-2859 of the sequence shown in SEQ ID NO: 1. As mentioned above, in addition to the GC content and/or number of ARFs, sequence optimization may also comprise a decrease in the number of CpG islands in the sequence and/or a decrease in the number of splice donor and acceptor sites. Of course, as is well known to those skilled in the art, sequence optimization is a balance between all these parameters, meaning that a sequence may be considered optimized if at least one of the above parameters is improved while one or more of the other parameters is not, as long as the optimized sequence leads to an improvement of the transgene, such as an improved expression and/or a decreased immune response to the transgene *in vivo.*

In addition, the adaptiveness of a nucleotide sequence encoding a functional GAA to the codon usage of human cells may be expressed as codon adaptation index (CAI). A codon adaptation index is herein defined as a measurement of the relative adaptiveness of the codon usage of a gene towards the codon usage of highly expressed human genes. The relative adaptiveness (w) of each codon is the ratio of the usage of each codon, to that of the most abundant codon for the same amino acid. The CAI is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li, 1987, Nucleic Acids Research 15: 1281-1295; also see: Kim et al, Gene. 1997, 199:293-301; zur Megede et al, Journal of Virology, 2000, 74: 2628-2635). Preferably, a nucleic acid molecule encoding a GAA has a CAI of at least 0.75 (in particular 0.77), 0.8, 0.85, 0.90, 0.92 or 0.94.

In one embodiment, the nucleic acid molecule of the invention encodes a protein having between 0 and 50, between 0 and 30, between 0 and 20, between 0 and 15, between 0 and 10, or between 0 and 5 amino acid changes to the protein encoded by the nucleotide sequence of SEQ ID NO: 13 or SEQ ID NO: 14. Furthermore, the GAA protein encoded by the nucleic acid of the invention may be a variant of a functional GAA protein known in the art, wherein the nucleic acid molecule of the invention encodes a protein having between 0 and 50, between 0 and 30, between 0 and 20, between 0 and 15, between 0 and 10, or between 0 and 5 amino acid changes to GAA protein known in the art. Such GAA protein known in the art that may serve as the basis for designing functional variant may be found in particular in the Uniprot entry of GAA (accession number P10253; corresponding to BenBank CAA68763.1; SEQ ID NO:37). In a further particular embodiment, the GAA moiety of the nucleic acid sequence of the invention encodes variants GAA polypeptides, or functional variants of such peptides as defined herein, such as those selected in the group consisting of the polypeptides identified as Genbank Accession Numbers AAA52506.1 (SEQ ID NO:38), EAW89583.1 (SEQ ID NO:39) and ABI53718.1 (SEQ ID NO:40). Other variant GAA polypeptides include those described in WO2012/145644, WO00/34451 and US6,858,425. In a particular embodiment, the parent GAA polypeptide is derived from the amino acid sequence shown in SEQ ID NO: 12 or SEQ ID NO:37.

In a particular embodiment, the GAA polypeptide encoded by the nucleic acid molecule of the invention is a functional GAA and has a sequence identity to hGAA protein shown in SEQ ID NO:5 or SEQ ID NO:36, in particular in SEQ ID NO:5, optionally taking into account the truncation carried out if a truncated form is considered as a reference to sequence identity, of at least 80 %, in particular at least 85 %, 90 %, 95 %, more particularly at least 96%, 97%, 98%, or 99%. In a particular embodiment, the GAA protein encoded by the nucleic acid molecule of the invention has the sequence shown in SEQ ID NO:5 or SEQ ID NO:36, in particular in SEQ ID NO:5.

The term "identical" and declinations thereof when referring to a polypeptide means that when a position in two compared polypeptide sequences is occupied by the same amino acid (e.g. if a position in each of two polypeptides is occupied by a leucine), then the polypeptides are identical at that position. The percent of identity between two polypeptides is a function of the number of matching positions shared by the two sequences divided by the number of positions compared X 100. For example, if 6 of 10 of the positions in two polypeptides are matched then the two sequences are 60% identical. Generally, a comparison is made when two sequences are aligned to give maximum identity. Various bioinformatic tools known to the one skilled in the art might be used to align nucleic acid sequences such as BLAST or FASTA.

The term "nucleic acid sequence" (or nucleic acid molecule) refers to a DNA or RNA molecule in single or double stranded form, particularly a DNA encoding a GAA protein according to the invention.

The invention also relates to a nucleic acid molecule encoding a chimeric functional GAA polypeptide comprising a signal peptide selected in the group consisting of SEQ ID NO:2 to 4.

In particular, the inventors have further surprisingly shown that signal peptide replacement results in the production of higher expression levels and higher secretion of functional GAA polypeptide as compared to a previously reported other chimeric GAA polypeptide comprising GAA fused to the signal peptide of human alpha-1-antitrypsin (hAAT, chimeric GAA protein described in WO2004064750 and Sun et al. 2006). In the nucleic acid molecule of the invention, the signal peptide moiety corresponds to a sequence encoding a signal peptide having an amino acid sequence selected in the group consisting of SEQ ID NO:2 to 4 (otherwise referred to herein as an "alternative signal peptide"). The nucleic acid molecule of the invention may further be an optimized sequence coding for a chimeric GAA polypeptide comprising an alternative signal peptide operably linked to a functional GAA polypeptide.

As compared to a wild-type GAA polypeptide, the endogenous signal peptide of wild-type GAA is replaced with an exogenous signal peptide, i.e. a signal peptide derived from a protein different from GAA. The exogenous signal peptide fused to the remainder of the GAA protein increases the secretion of the resulting chimeric GAA polypeptide as compared to the corresponding GAA polypeptide comprising its natural signal peptide. Furthermore, according to a particular embodiment of the invention, the nucleotide sequence corresponding to the alternative signal peptide may be an optimized sequence as provided above.

The signal peptides workable in the present invention include amino acids 1-25 from iduronate-2-sulphatase (SEQ ID NO:3), amino acids 1-20 from chymotrypsinogen B2 (SEQ ID NO:2) and amino acids 1-23 from protease C1 inhibitor (SEQ ID NO:4). The signal peptides of SEQ ID NO:2 to SEQ ID NO:4, allow higher secretion of the chimeric GAA protein both *in vitro* and *in vivo* when compared to the GAA comprising its natural signal peptide, or to a chimeric GAA protein comprising the signal peptide of hAAT.

The relative proportion of newly-synthesized GAA that is secreted from the cell can be routinely determined by methods known in the art and described in the examples. Secreted proteins can be detected by directly measuring the protein itself (e.g., by Western blot) or by protein activity assays (e.g., enzyme assays) in cell culture medium, serum, milk, etc.

Those skilled in the art will further understand that the chimeric GAA polypeptide can contain additional amino acids, e. g., as a result of manipulations of the nucleic acid construct such as the addition of a restriction site, as long as these additional amino acids do not render the signal peptide or the GAA polypeptide non-functional. The additional amino acids can be cleaved or can be retained by the mature polypeptide as long as retention does not result in a non-functional polypeptide.

Furthermore, the chimeric GAA polypeptide encoded by the nucleic acid molecule as herein described may comprise a GAA moiety that is a functional, truncated form of GAA. By "truncated form", it is meant a GAA polypeptide that comprises one or several consecutive amino acids deleted from the N-terminal part of a parent GAA polypeptide. Therefore, the GAA moiety in the chimeric GAA polypeptide of the invention may be a N-terminally truncated form of a parent GAA polypeptide. According to the present invention, a "parent GAA polypeptide" is a GAA polypeptide devoid of a signal peptide, such as a precursor form of a GAA devoid of a signal peptide, in particular the hGAA polypeptide shown in SEQ ID NO:5, or SEQ ID NO:36, in particular in SEQ ID NO5, and may be any of the variants as disclosed above. For example, with reference to typical wild-type human GAA polypeptides, the complete wild-type GAA polypeptide is represented in SEQ ID NO: 12 or in SEQ ID NO:37, and have a signal peptide, whereas the parent GAA polypeptide serving as basis for the truncated GAA form of this wild-type human GAA polypeptide is represented in SEQ ID NO:5 or SEQ ID NO:36, respectively, and have no signal peptide. In this example, the latter are referred to as a parent GAA polypeptide. In a variant of this particular embodiment, at least one amino acid is deleted from the N-terminal end of the parent GAA protein. In a particular embodiment, the GAA moiety may have at least 1, in particular at least 2, in particular at least 3, in particular at least 4, in particular at least 5, in particular at least 6, in particular at least 7, in particular at least 8 consecutive amino acids deleted from its N-terminal end as compared to the parent GAA polypeptide. For example, the GAA moiety may have 1 to 75 consecutive amino acids or more than 75 consecutive amino acids deleted from its N-terminal end as compared to the parent GAA polypeptide. In another embodiment, said GAA moiety has at most 75, in particular at most 70, in particular at most 60, in particular at most 55, in particular at most 50, in particular at most 47, in particular at most 46, in particular at most 45, in particular at most 44, in particular at most 43 consecutive amino acids deleted at its N-terminal end as compared to the parent GAA polypeptide. In a further particular embodiment, said GAA moiety has at most 47, in particular at most 46, in particular at most 45, in particular at most 44, in particular at most 43 consecutive amino acids deleted at its N-terminal end as compared to the parent GAA polypeptide. Specifically, the truncated GAA moiety may have 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74 or 75 consecutive amino acids deleted from its N-terminal end as compared to the parent GAA protein (in particular a truncated form of the parent hGAA polypeptide shown in SEQ ID NO:5 or SEQ ID NO:36, in particular in SEQ ID NO:5). In another particular embodiment, said GAA moiety has 1 to 75, in particular 1 to 47, in particular 1 to 46, in particular 1 to 45, in particular 1 to 44, in particular 1 to 43 consecutive amino acids deleted at its N-terminal end as compared to the parent GAA polypeptide. In another embodiment, said GAA moiety has 2 to 43, in particular 3 to 43, in particular 4 to 43, in particular 5 to 43, in particular 6 to 43, in particular 7 to 43, in particular 8 to 43 consecutive amino acids deleted at its N-terminal end as compared to the parent GAA polypeptide (in particular a truncated form of the parent hGAA polypeptide shown in SEQ ID NO:5 or SEQ ID NO:36, in particular in SEQ ID NO:5). Using an alternative nomenclature, the GAA polypeptide resulting from the truncation of 1 amino acid in the parent GAA polypeptide is referred to as Δ1 GAA truncated form, the GAA polypeptide resulting from the truncation of 2 consecutive amino acids from the N-terminal end is referred to as Δ2 GAA truncated form, the GAA polypeptide resulting from the truncation of 3 consecutive amino acids in the parent GAA polypeptide is referred to as Δ3 GAA truncated form), etc. In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46 or Δ47 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45 or Δ46 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44 or Δ45 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43 or Δ44 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the chimeric GAA protein of the invention comprises a Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42 or Δ43 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the GAA moiety of the chimeric GAA protein is a Δ6, Δ7, Δ8, Δ9 or Δ10 truncated form of GAA (in particular of the parent hGAA protein shown in SEQ ID NO: 5 or SEQ ID NO:36, in particular in SEQ ID NO:5), in particular a Δ7, Δ8 or Δ9 truncated form of GAA (in particular of the parent hGAA protein shown in SEQ ID NO: 5 or SEQ ID NO:36, in particular in SEQ ID NO:5), in particular a Δ8 truncated form of GAA (in particular of the parent hGAA protein shown in SEQ ID NO: 5 or SEQ ID NO:36, in particular in SEQ ID NO:5).

In a particular embodiment, the GAA moiety of the chimeric GAA protein is a Δ27, Δ28, Δ29, Δ30 or Δ31 truncated form of GAA (in particular of the parent hGAA protein shown in SEQ ID NO: 5 or SEQ ID NO:36, in particular in SEQ ID NO:5), in particular a Δ28, Δ29 or Δ30 truncated form of GAA (in particular of the parent hGAA protein shown in SEQ ID NO: 5 or SEQ ID NO:36, in particular in SEQ ID NO:5), in particular a Δ29 truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ40, Δ41, Δ42, Δ43 or Δ44 truncated form of GAA (in particular of the parent hGAA protein shown in SEQ ID NO: 5 or SEQ ID NO:36, in particular in SEQ ID NO:5), in particular a Δ41, Δ42 or Δ43 truncated form of GAA (in particular of the parent hGAA protein shown in SEQ ID NO: 5 or SEQ ID NO:36, in particular in SEQ ID NO:5), in particular a Δ42 truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ41, Δ42, Δ43, Δ44 or Δ45 truncated form of GAA (in particular of the parent hGAA protein shown in SEQ ID NO: 5 or SEQ ID NO:36, in particular in SEQ ID NO:5), in particular a Δ42, Δ43 or Δ44 truncated form of GAA (in particular of the parent hGAA protein shown in SEQ ID NO: 5 or SEQ ID NO:36, in particular in SEQ ID NO:5), in particular a Δ43 truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ6, Δ7, Δ8, Δ9, Δ10, Δ27, Δ28, Δ29, Δ30, Δ31, Δ40, Δ41, Δ42, Δ43, Δ44, Δ45, Δ46 or Δ47 truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ7, Δ8, Δ9, Δ28, Δ29, Δ30, Δ41, Δ42, Δ43 or Δ44 truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ6, Δ7, Δ8, Δ9, Δ10, Δ40, Δ41, Δ42, Δ43 or Δ44, truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ8, Δ29, Δ42, Δ43 or Δ47 truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ8, Δ29, Δ42 or Δ43 truncated form of GAA (in particular of the parent hGAA protein shown in SEQ ID NO: 5 or SEQ ID NO:36, in particular in SEQ ID NO:5).

In another particular embodiment, the GAA moiety of the chimeric GAA protein is a Δ8 or Δ42 truncated form of GAA (in particular of the parent hGAA protein shown in SEQ ID NO: 5 or SEQ ID NO:36, in particular in SEQ ID NO:5).

In a particular embodiment, of the invention, the chimeric GAA polypeptide of the invention comprises a truncated GAA moiety derived from a functional parent human GAA polypeptide. In a further particular embodiment, the parent hGAA polypeptide is the hGAA polypeptide shown in SEQ ID NO:5 or SEQ ID NO:36, in particular in SEQ ID NO:5. In a variant of this embodiment, the GAA moiety in the chimeric GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 75, 80, 85, 90, 91, 92, 93, 94, 95, 96, 97, 98 or 99 percent identity to SEQ ID NO:5 or SEQ ID NO:36, in particular to SEQ ID NO:5. In a further particular embodiment, the GAA moiety of the chimeric GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46 or Δ47, in particular a Δ6, Δ7, Δ8, Δ9, Δ10, Δ40, Δ41, Δ42, Δ43 or Δ44, in particular a Δ8, Δ29, Δ42 or Δ43, in particular a Δ8 or Δ42 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 75, 80, 85, 90, 91, 92, 93, 94, 95, 96, 97, 98 or 99 percent identity (for example 80, 85, 90, 95, 96, 97, 98 or 99 percent identity) to SEQ ID NO:5 or SEQ ID NO:36, in particular to SEQ ID NO:5.

In a variant of this embodiment, the GAA moiety of the chimeric GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45 or Δ46 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In a variant of this embodiment, the GAA moiety of the chimeric GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44 or Δ45 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43 or Δ44 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41 or Δ42 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, or Δ43 GAA truncated form of a hGAA polypeptide, and more particularly of the hGAA polypeptide shown in SEQ ID NO:5 or SEQ ID NO:36, even more particularly in SEQ ID NO:5, or of a functional variant thereof comprising amino acid substitutions in the sequence shown in SEQ ID NO:5 or SEQ ID NO:36, in particular SEQ ID NO:5, and having at least 75, 80, 85, 90, 91, 92, 93, 94, 95, 96, 97, 98 or 99 percent identity to SEQ ID NO:5 SEQ ID NO:36, in particular SEQ ID NO:5.

In another variant of this embodiment, the GAA moiety of the chimeric GAA polypeptide of the invention is a Δ6, Δ7, Δ8, Δ9 or Δ10, in particular a Δ7, Δ8 or Δ9, more particularly a Δ8 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ27, Δ28, Δ29, Δ30 or Δ31, in particular a Δ28, Δ29 or Δ30, more particularly a Δ29 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ40, Δ41, Δ42, Δ43 or Δ44, in particular a Δ41, Δ42 or Δ43, more particularly a Δ42 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ41, Δ42, Δ43, Δ44 or Δ45, in particular a Δ42, Δ43 or Δ44, more particularly a Δ43 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ6, Δ7, Δ8, Δ9, Δ10, Δ27, Δ28, Δ29, Δ30, Δ31, Δ40, Δ41, Δ42, Δ43, Δ44 or Δ45, in particular a Δ7, Δ8, Δ9, Δ28, Δ29, Δ30, Δ41, Δ42, Δ43 or Δ44, in particular a Δ8, Δ29, Δ42 or Δ43 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ6, Δ7, Δ8, Δ9, Δ10, Δ40, Δ41, Δ42, Δ43 or Δ44, in particular a Δ8 or Δ42 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ8, Δ29, Δ42, Δ43 or Δ47 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ8, Δ29, Δ42 or Δ43 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In another variant of this embodiment, the GAA moiety of the chimeric GAA polypeptide of the invention is a Δ8 or Δ42 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular in SEQ ID NO:5.

In a specific embodiment, the GAA moiety in the chimeric GAA polypeptide of the invention has an amino acid sequence consisting of the sequence shown in SEQ ID NO:29, SEQ ID NO:30, SEQ ID NO: 41, SEQ ID NO:42 or SEQ ID NO:43, in particular an amino acid sequences consisting of the sequence shown in SEQ ID NO:29, SEQ ID NO:30, SEQ ID NO: 41 or SEQ ID NO:42, in particular an amino acid sequences consisting of the sequence shown in SEQ ID NO:29 or SEQ ID NO:30.

The invention also relates to a nucleic acid construct comprising a nucleic acid molecule of the invention. The nucleic acid construct may correspond to an expression cassette comprising the nucleic acid sequence of the invention, operably linked to one or more expression control sequences and/or other sequences improving the expression of a transgene and/or sequences enhancing the secretion of the encoded protein and/or sequences enhancing the uptake of the encoded protein. As used herein, the term "operably linked" refers to a linkage of polynucleotide elements in a functional relationship. A nucleic acid is "operably linked" when it is placed into a functional relationship with another nucleic acid sequence. For instance, a promoter, or another transcription regulatory sequence, is operably linked to a coding sequence if it affects the transcription of the coding sequence. Such expression control sequences are known in the art, such as promoters, enhancers (such as cis-regulatory modules (CRMs)), introns, polyA signals, etc.

In particular, the expression cassette may include a promoter. The promoter may be an ubiquitous or tissue-specific promoter, in particular a promoter able to promote expression in cells or tissues in which expression of GAA is desirable such as in cells or tissues in which GAA expression is desirable in GAA-deficient patients. In a particular embodiment, the promoter is a liver-specific promoter such as the alpha-1 antitrypsin promoter (hAAT) (SEQ ID NO: 15), the transthyretin promoter, the albumin promoter, the thyroxine-binding globulin (TBG) promoter, the LSP promoter (comprising a thyroid hormone-binding globulin promoter sequence, two copies of an alpha1-microglobulin/bikunin enhancer sequence, and a leader sequence - 34.Ill, C. R., et al. (1997). Optimization of the human factor VIII complementary DNA expression plasmid for gene therapy of hemophilia A. Blood Coag. Fibrinol. 8: S23-S30.), etc. Other useful liver-specific promoters are known in the art, for example those listed in the Liver Specific Gene Promoter Database compiled the Cold Spring Harbor Laboratory (http://rulai.cshl.edu/LSPD/). A preferred promoter in the context of the invention is the hAAT promoter. In another embodiment, the promoter is a promoter directing expression in one tissue or cell of interest (such as in muscle cells), and in liver cells. For example, to some extent, promoters specific of muscle cells such as the desmin, Spc5-12 and MCK promoters may present some leakage of expression into liver cells, which can be advantageous to induce immune tolerance of the subject to the GAA protein expressed from the nucleic acid of the invention.

Other tissue-specific or non-tissue-specific promoters may be useful in the practice of the invention. For example, the expression cassette may include a tissue-specific promoter which is a promoter different from a liver specific promoter. For example the promoter may be muscle-specific, such as the desmin promoter (and a desmin promoter variant such as a desmin promoter including natural or artificial enhancers), the SPc5-12 promoter or the MCK promoter. In another embodiment, the promoter is a promoter specific of other cell lineage, such as the erythropoietin promoter, for the expression of the GAA polypeptide from cells of the erythroid lineage.

In another embodiment, the promoter is an ubiquitous promoter. Representative ubiquitous promoters include the cytomegalovirus enhancer/chicken beta actin (CAG) promoter, the cytomegalovirus enhancer/promoter (CMV), the PGK promoter, the SV40 early promoter, etc.

In addition, the promoter may also be an endogenous promoter such as the albumin promoter or the GAA promoter.

In a particular embodiment, the promoter is associated to an enhancer sequence, such as cis-regulatory modules (CRMs) or an artificial enhancer sequence. For example, the promoter may be associated to an enhancer sequence such as the human ApoE control region (or Human apolipoprotein E/C-I gene locus, hepatic control region HCR-1 - Genbank accession No. U32510, shown in SEQ ID NO:16). In a particular embodiment, an enhancer sequence such as the ApoE sequence is associated to a liver-specific promoter such as those listed above, and in particular such as the hAAT promoter. Other CRMs useful in the practice of the present invention include those described in Rincon et al., Mol Ther. 2015 Jan;23(1):43-52, Chuah et al., Mol Ther. 2014 Sep;22(9):1605-13 or Nair et al., Blood. 2014 May 15;123(20):3195-9.

In another particular embodiment, the nucleic acid construct comprises an intron, in particular an intron placed between the promoter and the GAA coding sequence. An intron may be introduced to increase mRNA stability and the production of the protein. In a further embodiment, the nucleic acid construct comprises a human beta globin b2 (or HBB2) intron, a coagulation factor IX (FIX) intron, a SV40 intron or a chicken beta-globin intron. In another further embodiment, the nucleic acid construct of the invention contains a modified intron (in particular a modified HBB2 or FIX intron) designed to decrease the number of, or even totally remove, alternative open reading frames (ARFs) found in said intron. Preferably, ARFs are removed whose length spans over 50 bp and have a stop codon in frame with a start codon. ARFs may be removed by modifying the sequence of the intron. For example, modification may be carried out by way of nucleotide substitution, insertion or deletion, preferably by nucleotide substitution. As an illustration, one or more nucleotides, in particular one nucleotide, in an ATG or GTG start codon present in the sequence of the intron of interest may be replaced resulting in a non-start codon. For example, an ATG or a GTG may be replaced by a CTG, which is not a start codon, within the sequence of the intron of interest.

The classical HBB2 intron used in nucleic acid constructs is shown in SEQ ID NO:6. For example, this HBB2 intron may be modified by eliminating start codons (ATG and GTG codons) within said intron. In a particular embodiment, the modified HBB2 intron comprised in the construct has the sequence shown in SEQ ID NO:7. The classical FIX intron used in nucleic acid constructs is derived from the first intron of human FIX and is shown in SEQ ID NO:8. FIX intron may be modified by eliminating start codons (ATG and GTG codons) within said intron. In a particular embodiment, the modified FIX intron comprised in the construct of the invention has the sequence shown in SEQ ID NO:9. The classical chicken-beta globin intron used in nucleic acid constructs is shown in SEQ ID NO: 10. Chicken-beta globin intron may be modified by eliminating start codons (ATG and GTG codons) within said intron. In a particular embodiment, the modified chicken-beta globin intron comprised in the construct of the invention has the sequence shown in SEQ ID NO:11.

The inventors have previously shown in WO2015/162302 that such a modified intron, in particular a modified HBB2 or FIX intron, has advantageous properties and can significantly improve the expression of a transgene.

In a particular embodiment, the nucleic acid construct of the invention is an expression cassette comprising, in the 5' to 3' orientation, a promoter optionally preceded by an enhancer, the coding sequence of the invention (i.e. the optimized GAA coding sequence of the invention, the chimeric GAA coding sequence of the invention, or the chimeric and optimized GAA coding sequence of the invention), and a polyadenylation signal (such as the bovine growth hormone polyadenylation signal, the SV40 polyadenylation signal, or another naturally occurring or artificial polyadenylation signal). In a particular embodiment, the nucleic acid construct of the invention is an expression cassette comprising, in the 5' to 3' orientation, a promoter optionally preceded by an enhancer, (such as the ApoE control region), an intron (in particular an intron as defined above), the coding sequence of the invention, and a polyadenylation signal. In a further particular embodiment, the nucleic acid construct of the invention is an expression cassette comprising, in the 5' to 3' orientation, an enhancer such as the ApoE control region, a promoter, an intron (in particular an intron as defined above), the coding sequence of the invention, and a polyadenylation signal. In a further particular embodiment of the invention the expression cassette comprising, in the 5' to 3' orientation, an ApoE control region, the hAAT-liver specific promoter, a HBB2 intron (in particular a modified HBB2 intron as defined above), the coding sequence of the invention, and the bovine growth hormone polyadenylation signal, such as the nucleic acid construct shown in any one of SEQ ID NO:20 to SEQ ID NO:22, which includes the sequence-optimized GAA nucleic acid molecule of SEQ ID NO: 13 combined to each of the signal peptideencoding sequences shown in SEQ ID NO:2 to 4. In other embodiments, the expression cassette contains the coding sequence resulting from one of the combinations of sequences shown in table 2, table 2" or table 2" above, in particular in table 2' or table 2".

In a particular embodiment, the expression cassette comprises the ApoE control region, the hAAT-liver specific promoter, a codon-optimized HBB2 intron, the coding sequence of the invention and the bovine growth hormone polyadenylation signal.

In designing the nucleic acid construct of the invention, one skilled in the art will take care of respecting the size limit of the vector used for delivering said construct to a cell or organ. In particular, one skilled in the art knows that a major limitation of AAV vector is its cargo capacity which may vary from one AAV serotype to another but is thought to be limited to around the size of parental viral genome. For example, 5 kb, is the maximum size usually thought to be packaged into an AAV8 capsid (Wu Z. et al., Mol Ther., 2010, 18(1): 80-86; Lai Y. et al., Mol Ther., 2010, 18(1): 75-79; Wang Y. et al., Hum Gene Ther Methods, 2012, 23(4): 225-33). Accordingly, those skilled in the art will take care in practicing the present invention to select the components of the nucleic acid construct of the invention so that the resulting nucleic acid sequence, including sequences coding AAV 5'- and 3'-ITRs to preferably not exceed 110 % of the cargo capacity of the AAV vector implemented, in particular to preferably not exceed 5.5 kb.

It is also herein disclosed a vector comprising a nucleic acid molecule or construct as disclosed herein. In particular, the vector is a vector suitable for protein expression, preferably for use in gene therapy. In one embodiment, the vector is a plasmid vector. In another embodiment, the vector is a nanoparticle containing a nucleic acid molecule of the invention, in particular a messenger RNA encoding the GAA polypeptide of the invention. In another embodiment, the vector is a system based on transposons, allowing integration of the nucleic acid molecule or construct of the invention in the genome of the target cell, such as the hyperactive Sleeping Beauty (SB100X) transposon system (Mates et al. 2009). In another embodiment, the vector is a viral vector suitable for gene therapy, targeting any cell of interest such as liver tissue or cells, muscle cell, CNS cells (such as brain cells), or hematopoietic stem cells such as cells of the erythroid lineage (such as erythrocytes). In this case, the nucleic acid construct of the invention also contains sequences suitable for producing an efficient viral vector, as is well known in the art. In a particular embodiment, the viral vector is derived from an integrating virus. In particular, the viral vector may be derived from a retrovirus or a lentivirus. In a further particular embodiment, the viral vector is an AAV vector, such as an AAV vector suitable for transducing liver tissues or cells, more particularly an AAV-1, -2 and AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p. 16026), -7, -8, -9, -10 such as -cy10 and -rh10, -rh74, -dj, Anc80, LK03, AAV2i8, porcine AAV serotypes such as AAVpo4 and AAVpo6, etc., vector or a retroviral vector such as a lentiviral vector and an alpha-retrovirus. As is known in the art, depending on the specific viral vector considered for use, additional suitable sequences will be introduced in the nucleic acid construct of the invention for obtaining a functional viral vector. Suitable sequences include AAV ITRs for an AAV vector, or LTRs for lentiviral vectors. As such, the invention also relates to an expression cassette as described above, flanked by an ITR or an LTR on each side.

Advantages of viral vectors are discussed in the following part of this disclosure. Viral vectors are preferred for delivering the nucleic acid molecule or construct of the invention, such as a retroviral vector, for example a lentiviral vector, or a non-pathogenic parvovirus, more preferably an AAV vector. The human parvovirus Adeno-Associated Virus (AAV) is a dependovirus that is naturally defective for replication which is able to integrate into the genome of the infected cell to establish a latent infection. The last property appears to be unique among mammalian viruses because the integration occurs at a specific site in the human genome, called AAVS1, located on chromosome 19 (19q13.3-qter). Therefore, AAV vectors have arisen considerable interest as a potential vectors for human gene therapy. Among the favorable properties of the virus are its lack of association with any human disease, its ability to infect both dividing and non-dividing cells, and the wide range of cell lines derived from different tissues that can be infected.

Among the serotypes of AAVs isolated from human or non-human primates (NHP) and well characterized, human serotype 2 is the first AAV that was developed as a gene transfer vector. Other currently used AAV serotypes include AAV-1, AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p. 16026), -7, -8, -9, -10 such as cy10 and -rh10, -rh74, -dj, Anc80, LK03, AAV2i8, porcine AAV serotypes such as AAVpo4 and AAVpo6, and tyrosine, lysine and serine capsid mutants of the AAV serotypes, etc.. In addition, other non-natural engineered variants and chimeric AAV can also be useful.

AAV viruses may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus.

Desirable AAV fragments for assembly into vectors include the cap proteins, including the vp1, vp2, vp3 and hypervariable regions, the rep proteins, including rep 78, rep 68, rep 52, and rep 40, and the sequences encoding these proteins. These fragments may be readily utilized in a variety of vector systems and host cells.

AAV-based recombinant vectors lacking the Rep protein integrate with low efficacy into the host's genome and are mainly present as stable circular episomes that can persist for years in the target cells. Alternatively to using AAV natural serotypes, artificial AAV serotypes may be used in the context of the present disclosure, including, without limitation, AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid, a recombinant AAV capsid, or a "humanized" AAV capsid. Accordingly, it is herein disclosed an AAV vector comprising the nucleic acid molecule or construct of the invention. In the context of the present disclosure, the AAV vector comprises an AAV capsid able to transduce the target cells of interest, in particular hepatocytes. According to a particular embodiment, the AAV vector is of the AAV-1, -2, AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p. 16026), -7, -8, -9, -10 such as -cy10 and -rh10, -rh74, -dj, Anc80, LK03, AAV2i8, porcine AAV such as AAVpo4 and AAVpo6, and tyrosine, lysine and serine capsid mutants of a AAV serotypes, etc., serotype. In a particular embodiment, the AAV vector is of the AAV8, AAV9, AAVrh74 or AAV2i8 serotype (i.e. the AAV vector has a capsid of the AAV8, AAV9, AAVrh74 or AAV2i8 serotype). In a further particular embodiment, the AAV vector is a pseudotyped vector, i.e. its genome and capsid are derived from AAVs of different serotypes. For example, the pseudotyped AAV vector may be a vector whose genome is derived from one of the above mentioned AAV serotypes, and whose capsid is derived from another serotype. For example, the genome of the pseudotyped vector may have a capsid derived from the AAV8, AAV9, AAVrh74 or AAV2i8 serotype, and its genome may be derived from and different serotype. In a particular embodiment, the AAV vector has a capsid of the AAV8, AAV9 or AAVrh74 serotype, in particular of the AAV8 or AAV9 serotype, more particularly of the AAV8 serotype.

In a specific embodiment, wherein the vector is for use in delivering the transgene to muscle cells, the AAV vector may be selected, among others, in the group consisting of AAV8, AAV9 and AAVrh74. In another specific embodiment, wherein the vector is for use in delivering the transgene to liver cells, the AAV vector may be selected, among others, in the group consisting of AAV5, AAV8, AAV9, AAV-LK03, AAV-Anc80 and AAV3B.

In another embodiment, the capsid is a modified capsid. In the context of the present disclosure, a "modified capsid" may be a chimeric capsid or capsid comprising one or more variant VP capsid proteins derived from one or more wild-type AAV VP capsid proteins.

In a particular embodiment, the AAV vector is a chimeric vector, i.e. its capsid comprises VP capsid proteins derived from at least two different AAV serotypes, or comprises at least one chimeric VP protein combining VP protein regions or domains derived from at least two AAV serotypes. Examples of such chimeric AAV vectors useful to transduce liver cells are described in Shen et al., Molecular Therapy, 2007 and in Tenney et al., Virology, 2014. For example a chimeric AAV vector can derive from the combination of an AAV8 capsid sequence with a sequence of an AAV serotype different from the AAV8 serotype, such as any of those specifically mentioned above. In another embodiment, the capsid of the AAV vector comprises one or more variant VP capsid proteins such as those described in WO2015013313, in particular the RHM4-1, RHM15-1, RHM15-2, RHM15-3/RHM15-5, RHM15-4 and RHM15-6 capsid variants, which present a high liver tropism.

In another embodiment, the modified capsid can be derived also from capsid modifications inserted by error prone PCR and/or peptide insertion (e.g. as described in Bartel et al., 2011). In addition, capsid variants may include single amino acid changes such as tyrosine mutants (e.g. as described in Zhong et al., 2008)

In addition, the genome of the AAV vector may either be a single stranded or self-complementary double-stranded genome (McCarty et al., Gene Therapy, 2003). Self-complementary double-stranded AAV vectors are generated by deleting the terminal resolution site (trs) from one of the AAV terminal repeats. These modified vectors, whose replicating genome is half the length of the wild type AAV genome have the tendency to package DNA dimers. In a preferred embodiment, the AAV vector implemented in the practice of the present disclosure has a single stranded genome, and further preferably comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid.

In a particularly preferred embodiment, it is herein disclosed an AAV vector comprising, in a single-stranded or double-stranded, self-complementary genome (e.g. a single-stranded genome), the nucleic acid acid construct of the invention. In one embodiment, the AAV vector comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid . In a further particular embodiment, said nucleic acid is operably linked to a promoter, especially an ubiquitous or liver-specific promoter. According to a specific variant embodiment, the promoter is an ubiquitous promoter such as the cytomegalovirus enhancer/chicken beta actin (CAG) promoter, the cytomegalovirus enhancer/promoter (CMV), the PGK promoter and the SV40 early promoter. In a specific variant, the ubiquitous promoter is the CAG promoter. According to another variant, the promoter is a liver-specific promoter such as the alpha-1 antitrypsin promoter (hAAT), the transthyretin promoter, the albumin promoter and the thyroxine-binding globulin (TBG) promoter. In a specific variant, the liver-specific promoter is the hAAT liver-specific promoter of SEQ ID NO: 15. In a further particular embodiment, the nucleic acid construct comprised into the genome of the AAV vector of the present disclosure further comprises an intron as described above, such as an intron placed between the promoter and the nucleic acid sequence encoding the GAA coding sequence (i.e. the optimized GAA coding sequence of the invention, the chimeric GAA coding sequence of the invention, or the chimeric and optimized GAA coding sequence of the invention). Representative introns that may be included within the nucleic acid construct introduced within the AAV vector genome include, without limitation, the human beta globin b2 (or HBB2) intron, the FIX intron and the chicken beta-globin intron. Said intron within the genome of the AAV vector may be a classical (or unmodified) intron or a modified intron designed to decrease the number of, or even totally remove, alternative open reading frames (ARFs) within said intron. Modified and unmodified introns that may be used in the practice of this embodiment where the nucleic acid of the invention is introduced within an AAV vector are thoroughly described above. In a particular embodiment, the AAV vector, in particular an AAV vector comprising an AAV8, AAV9, AAVrh74 or AAV2i8 capsid, in particular an AAV8, AAV9 or AAVrh74 capsid, such as an AAV8 or AAV9 capsid, more particularly an AAV8 capsid, of the present disclosure includes within its genome a modified (or optimized) intron such as the modified HBB2 intron of SEQ ID NO:7, the modified FIX intron of SEQ ID NO:9 and the modified chicken beta-globin intron of SEQ ID NO:11. In a further particular embodiment, the vector of the present disclosure is an AAV vector comprising comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid, comprising a genome containing, in the 5' to 3' orientation: an AAV 5'-ITR (such as an AAV2 5'-ITR); an ApoE control region; the hAAT-liver specific promoter; a HBB2 intron (in particular a modified HBB2 intron as defined above); the GAA coding sequence of the invention; the bovine growth hormone polyadenylation signal; and an AAV 3'-ITR (such as an AAV2 3'-ITR), such as a genome comprising a the nucleic acid shown in SEQ ID NO:20, 21 or 22 (including the nucleic acid sequence shown in SEQ ID NO:17, 18 and 19, respectively, corresponding to an optimized sequence encoding a Δ8 truncated form of GAA derived from the parent hGAA of SEQ ID NO:5) flanked by an AAV 5'-ITR (such as an AAV2 5'-ITR) and an AAV 3'-ITR (such as an AAV2 3'-ITR). Other expression cassette useful in the practice of the present disclosure comprise those signal peptide moiety and GAA moiety in any one of the sequence combinations shown in table 2, table 2' or table 2", in particular in table 2' or table 2" above.

In a particular embodiment of the invention, the nucleic acid construct of the invention comprises a liver-specific promoter as described above, and the vector is a viral vector capable of transducing liver tissue or cells as described above. The inventors present below data showing that the protolerogenic and metabolic properties of the liver are advantageously implemented thanks to this embodiment to develop highly efficient and optimized vectors to express secretable forms of GAA in hepatocytes and to induce immune tolerance to the protein.

In addition, in a further particular embodiment, the present disclosure provides the combination of two vectors, such as two viral vectors, in particular two AAV vectors, for improving gene delivery and treatment efficacy in the cells of interest. For example, the two vectors may carry the nucleic acid molecule of the invention coding for the GAA protein of the invention, under the control of one different promoter in each of these two vectors. In a particular embodiment, one vector comprises a promoter which is a liver-specific promoter (as one of those described above), and the other vector comprises a promoter which is specific of another tissue of interest for the treatment of a glycogen storage disorder, such as a muscle-specific promoter, for example the desmin promoter. In a particular variant of this embodiment, this combination of vectors corresponds to multiple co-packaged AAV vectors produced as described in WO2015196179.

In another aspect, the invention provides a chimeric GAA polypeptide, comprising a signal peptide moiety and a GAA moiety, wherein the naturally occurring GAA signal peptide is replaced with a signal peptide selected in the group consisting of SEQ ID NO:2 to 4. In a particular embodiment, the chimeric GAA polypeptide of the invention may be a polypeptide derived from a truncated form of GAA, as described above. For example, the chimeric GAA protein of the invention may a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4.

In a particular embodiment, the GAA moiety of the chimeric GAA protein is a Δ6, Δ7, Δ8, Δ9 or Δ10 truncated form of GAA (in particular of the parent hGAA protein shown in SEQ ID NO: 5 or SEQ ID NO:36, in particular in SEQ ID NO:5), in particular a Δ7, Δ8 or Δ9 truncated form of GAA (in particular of the parent hGAA protein shown in SEQ ID NO: 5 or SEQ ID NO:36, in particular in SEQ ID NO:5), in particular a Δ8 truncated form of GAA (in particular of the parent hGAA protein shown in SEQ ID NO: 5 or SEQ ID NO:36, in particular in SEQ ID NO:5).

In another particular embodiment, the truncated GAA polypeptide of the invention is a Δ27, Δ28, Δ29, Δ30 or Δ31, in particular a Δ28, Δ29 or Δ30, more particularly a Δ29 truncated form of a hGAA polypeptide, and more particularly of the hGAA polypeptide shown in SEQ ID NO: 1 or SEQ ID NO:33, in particular in SEQ ID NO: 1, or of a functional variant thereof comprising amino acid substitutions in the sequence shown in SEQ ID NO: 1 or SEQ ID NO:33, in particular in SEQ ID NO: 1, and having at least 80, 85, 90, 95, 96, 97, 98 or 99 percent identity to SEQ ID NO:1 or SEQ ID NO:33, in particular in SEQ ID NO:1.

In another particular embodiment, the GAA moiety of the chimeric GAA protein is a Δ40, Δ41, Δ42, Δ43 or Δ44 truncated form of GAA (in particular of the parent hGAA protein shown in SEQ ID NO: 5 or SEQ ID NO:36, in particular in SEQ ID NO:5), in particular a Δ41, Δ42 or Δ43 truncated form of GAA (in particular of the parent hGAA protein shown in SEQ ID NO: 5 or SEQ ID NO:36, in particular in SEQ ID NO:5), in particular a Δ42 truncated form of GAA (in particular of the parent hGAA protein shown in SEQ ID NO: 5 or SEQ ID NO:36, in particular in SEQ ID NO:5).

In another variant of this embodiment, the truncated GAA polypeptide of the invention is a Δ41, Δ42, Δ43, Δ44 or Δ45, in particular a Δ42, Δ43 or Δ44, more particularly a Δ43 truncated form of a hGAA polypeptide, and more particularly of the hGAA polypeptide shown in SEQ ID NO: 1 or SEQ ID NO:33, in particular in SEQ ID NO: 1, or of a functional variant thereof comprising amino acid substitutions in the sequence shown in SEQ ID NO: 1 or SEQ ID NO:33, in particular in SEQ ID NO: 1, and having at least 80, 85, 90, 95, 96, 97, 98 or 99 percent identity to SEQ ID NO:1 or SEQ ID NO:33, in particular in SEQ ID NO:1.

In another variant of this embodiment, the truncated GAA polypeptide of the invention is a Δ6, Δ7, Δ8, Δ9, Δ10, Δ27, Δ28, Δ29, Δ30, Δ31, Δ40, Δ41, Δ42, Δ43, Δ44 or Δ45, in particular a Δ7, Δ8, Δ9, Δ28, Δ29, Δ30, Δ41, Δ42, Δ43 or Δ44, in particular a Δ8, Δ29, Δ42 or Δ43 truncated form of a hGAA polypeptide, and more particularly of the hGAA polypeptide shown in SEQ ID NO: 1 or SEQ ID NO:33, in particular in SEQ ID NO: 1, or of a functional variant thereof comprising amino acid substitutions in the sequence shown in SEQ ID NO: 1 or SEQ ID NO:33, in particular in SEQ ID NO: 1, and having at least 80, 85, 90, 95, 96, 97, 98 or 99 percent identity to SEQ ID NO:1 or SEQ ID NO:33, in particular in SEQ ID NO:1.

In another variant of this embodiment, the truncated GAA polypeptide of the invention is a Δ6, Δ7, Δ8, Δ9, Δ10, Δ40, Δ41, Δ42, Δ43 or Δ44, in particular a Δ8 or Δ42 truncated form of a hGAA polypeptide, and more particularly of the hGAA polypeptide shown in SEQ ID NO: 1 or SEQ ID NO:33, in particular in SEQ ID NO:1, or of a functional variant thereof comprising amino acid substitutions in the sequence shown in SEQ ID NO:1 or SEQ ID NO:33, in particular in SEQ ID NO:1, and having at least 80, 85, 90, 95, 96, 97, 98 or 99 percent identity to SEQ ID NO:1 or SEQ ID NO:33, in particular in SEQ ID NO:1.

In another variant of this embodiment, the truncated GAA polypeptide of the invention is a Δ8, Δ29, Δ42, Δ43 or Δ47 truncated form of a hGAA polypeptide, and more particularly of the hGAA polypeptide shown in SEQ ID NO:1 or SEQ ID NO:33, in particular in SEQ ID NO:1, or of a functional variant thereof comprising amino acid substitutions in the sequence shown in SEQ ID NO:1 or SEQ ID NO:33, in particular in SEQ ID NO:1, and having at least 80, 85, 90, 95, 96, 97, 98 or 99 percent identity to SEQ ID NO:1 or SEQ ID NO:33, in particular in SEQ ID NO: 1.

In another variant of this embodiment, the truncated GAA polypeptide of the invention is a Δ8, Δ29, Δ42 or Δ43 truncated form of a hGAA polypeptide, and more particularly of the hGAA polypeptide shown in SEQ ID NO:1 or SEQ ID NO:33, in particular in SEQ ID NO:1, or of a functional variant thereof comprising amino acid substitutions in the sequence shown in SEQ ID NO:1 or SEQ ID NO:33, in particular in SEQ ID NO:1, and having at least 80, 85, 90, 95, 96, 97, 98 or 99 percent identity to SEQ ID NO:1 or SEQ ID NO:33, in particular in SEQ ID NO: 1.

In another variant of this embodiment, the truncated GAA polypeptide of the invention is a Δ8 or Δ42 truncated form of a hGAA polypeptide, and more particularly of the hGAA polypeptide shown in SEQ ID NO: 1 or SEQ ID NO:33, in particular in SEQ ID NO:1, or of a functional variant thereof comprising amino acid substitutions in the sequence shown in SEQ ID NO: 1 or SEQ ID NO:33, in particular in SEQ ID NO: 1, and having at least 80, 85, 90, 95, 96, 97, 98 or 99 percent identity to SEQ ID NO: 1 or SEQ ID NO:33, in particular in SEQ ID NO: 1.

In a specific embodiment, the truncated hGAA polypeptide of the invention has an amino acid sequence consisting of the sequence shown in SEQ ID NO:29, SEQ ID NO:30, SEQ ID NO:41, SEQ ID NO:42 or SEQ ID NO:43, or a functional variant thereof comprising from 1 to 5 amino, in particular from 1 to 4, in particular from 1 to 3, more particularly from 1 to 2, in particular 1 amino acid substitution as compared to the sequence shown in SEQ ID NO:29, SEQ ID NO:30, SEQ ID NO:41, SEQ ID NO:42 or SEQ ID NO:43. In another specific embodiment, the truncated hGAA polypeptide of the invention has an amino acid sequence consisting of the sequence shown in SEQ ID NO:29, SEQ ID NO:30, SEQ ID NO:41 or SEQ ID NO:42, or a functional variant thereof comprising from 1 to 5 amino acid substitutions as compared to the sequence shown in SEQ ID NO:29, SEQ ID NO:30, SEQ ID NO:41 or SEQ ID NO:42. In a specific embodiment, the truncated hGAA polypeptide of the invention has an amino acid sequence consisting of the sequence shown in SEQ ID NO:29 or SEQ ID NO:30, or a functional variant thereof comprising from 1 to 5 amino, in particular from 1 to 4, in particular from 1 to 3, more particularly from 1 to 2, in particular 1 amino acid substitution as compared to the sequence shown in SEQ ID NO:29 or SEQ ID NO:30.

In a particular embodiment, the chimeric GAA polypeptide has the sequence resulting from one of the combination shown in table 1, table 1' or table 1" above, in particular in table 1' or table 1",, or is a functional derivative thereof having at least 90% identity, in particular at least 95%, at least 96%, at least 97%, at least 98%, or at least 99% identity to the resulting sequence combination.

The invention also relates to a cell, for example a liver cell, that is transformed with a nucleic acid molecule or construct of the invention as is the case for *ex vivo* gene therapy. Cells of the invention may be delivered to the subject in need thereof, such as GAA-deficient patient, by any appropriate administration route such as via injection in the liver or in the bloodstream of said subject. In a particular embodiment, the invention involves introducing the nucleic acid of the invention into liver cells, in particular into liver cells of the subject to be treated, and administering said transformed liver cells into which the nucleic acid has been introduced to the subject. Advantageously, this embodiment is useful for secreting GAA from said cells. In a particular embodiment, the liver cells are liver cells from the patient to be treated, or are liver stem cells that are further transformed, and differentiated *in vitro* into liver cells, for subsequent administration to the patient.

The present invention further relates to a transgenic, nonhuman animal comprising in its genome the nucleic acid molecule or construct encoding a GAA protein according to the invention. In a particular embodiment, the animal is a mouse.

Apart from the specific delivery systems embodied below in the examples, various delivery systems are known and can be used to administer the nucleic acid molecule or construct of the invention, e.g., encapsulation in liposomes, microparticles, microcapsules, recombinant cells capable of expressing the coding sequence of the invention, receptor-mediated endocytosis, construction of a therapeutic nucleic acid as part of a retroviral or other vector, etc.

According to an embodiment, it may be desirable to introduce the chimeric GAA polypeptide, nucleic acid molecule, nucleic acid construct or cell of the invention into the liver of the subject by any suitable route. In addition naked DNA such as minicircles and transposons can be used for delivery or lentiviral vectors. Additionally, gene editing technologies such as zinc finger nucleases, meganucleases, TALENs, and CRISPR can also be used to deliver the coding sequence of the invention.

The present invention also provides pharmaceutical compositions comprising the nucleic acid molecule, the nucleic acid construct, the chimeric GAA polypeptide, or the cell of the invention. The present disclosure also provides a pharmaceutical composition comprising the vector disclosed herein. Such compositions comprise a therapeutically effective amount of the therapeutic (the nucleic acid molecule, the nucleic acid construct, the chimeric GAA polypeptide or the cell of the invention, or the vector of the present disclosure), and a pharmaceutically acceptable carrier. In a specific embodiment, the term "pharmaceutically acceptable" means approved by a regulatory agency of the Federal or a state government or listed in the U.S. or European Pharmacopeia or other generally recognized pharmacopeia for use in animals, and humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsions, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the subject. In a particular embodiment, the nucleic acid or cell of the invention, or the vector herein disclosed, is formulated in a composition comprising phosphate-buffered saline and supplemented with 0.25% human serum albumin. In another particular embodiment, the nucleic acid, or cell of the invention, or the vector herein disclosed, is formulated in a composition comprising ringer lactate and a non-ionic surfactant, such as pluronic F68 at a final concentration of 0.01-0.0001%, such as at a concentration of 0.001%, by weight of the total composition. The formulation may further comprise serum albumin, in particular human serum albumin, such as human serum albumin at 0.25%. Other appropriate formulations for either storage or administration are known in the art, in particular from WO 2005/118792 or Allay et al., 2011.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection.

In an embodiment, the nucleic acid molecule, the nucleic acid construct, the chimeric GAA polypeptide or the cell of the invention, or the vector herein disclosed, can be delivered in a vesicle, in particular a liposome. In yet another embodiment, the nucleic acid molecule, the nucleic acid construct, the chimeric GAA polypeptide or the cell of the invention, or the vector herein disclosed, can be delivered in a controlled release system.

Methods of administration of the nucleic acid molecule, the nucleic acid construct, the chimeric GAA polypeptide or the cell of the invention, or the vector herein disclosed, include but are not limited to intradermal, intramuscular, intraperitoneal, intravenous, subcutaneous, intranasal, epidural, and oral routes. In a particular embodiment, the administration is via the intravenous or intramuscular route. The nucleic acid molecule, the nucleic acid construct, the chimeric GAA polypeptide or the cell of the invention, , or the vector herein disclosed, whether vectorized or not, may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

In a specific embodiment, it may be desirable to administer the pharmaceutical compositions of the invention locally to the area in need of treatment, e.g. the liver. This may be achieved, for example, by means of an implant, said implant being of a porous, nonporous, or gelatinous material, including membranes, such as sialastic membranes, or fibers.

The amount of the therapeutic (i.e. the nucleic acid molecule, the nucleic acid construct, the chimeric GAA polypeptide or the cell of the invention) of the invention, or the amount of the vector herein disclosed, which will be effective in the treatment of a glycogen storage disease can be determined by standard clinical techniques. In addition, in vivo and/or in vitro assays may optionally be employed to help predict optimal dosage ranges. The precise dose to be employed in the formulation will also depend on the route of administration, and the seriousness of the disease, and should be decided according to the judgment of the practitioner and each patient's circumstances. The dosage of the nucleic acid molecule, the nucleic acid construct, the chimeric GAA polypeptide or the cell of the invention, or of the vector herein disclosed, administered to the subject in need thereof will vary based on several factors including, without limitation, the route of administration, the specific disease treated, the subject's age or the level of expression necessary to obtain the therapeutic effect. One skilled in the art can readily determine, based on its knowledge in this field, the dosage range required based on these factors and others. In case of a treatment comprising administering a viral vector, such as an AAV vector, to the subject, typical doses of the vector are of at least 1×10⁸ vector genomes per kilogram body weight (vg/kg), such as at least 1×10⁹ vg/kg, at least 1×10¹⁰ vg/kg, at least 1×10¹¹ vg/kg, at least 1×10¹² vg/kg at least 1×10¹³ vg/kg, or at least 1×10¹⁴ vg/kg.

It is also herein disclosed a method for treating a glycogen storage disease, which comprises a step of delivering a therapeutic effective amount of the nucleic acid, the chimeric polypeptide, the pharmaceutical composition or the cell of the invention, or of the vector herein disclosed, to a subject in need thereof.

It is also herein disclosed a method for treating a glycogen storage disease, said method inducing no immune response to the transgene (i.e. to the chimeric GAA polypeptide of the invention), or inducing a reduced immune response to the transgene, comprising a step of delivering a therapeutic effective amount of the nucleic acid molecule, nucleic acid construct, pharmaceutical composition or cell of the invention, or of the vector herein disclosed, to a subject in need thereof. It is also herein disclosed a method for treating a glycogen storage disease, said method comprising repeated administration of a therapeutic effective amount of the nucleic acid molecule, nucleic acid construct, pharmaceutical composition or cell of the invention, or of the vector herein disclosed, to a subject in need thereof. In this aspect, the nucleic acid molecule or the nucleic acid construct of the invention comprises a promoter which is functional in liver cells, thereby allowing immune tolerance to the expressed chimeric GAA polypeptide produced therefrom. As well, in this aspect, the pharmaceutical composition used in this aspect comprises a nucleic acid molecule or nucleic acid construct comprising a promoter which is functional in liver cells. In case of delivery of liver cells, said cells may be cells previously collected from the subject in need of the treatment and that were engineered by introducing therein the nucleic acid molecule or the nucleic acid construct of the invention to thereby make them able to produce the chimeric GAA polypeptide of the invention. According to an embodiment, in the aspect comprising a repeated administration, said administration may be repeated at least once or more, and may even be considered to be done according to a periodic schedule, such as once per week, per month or per year. The periodic schedule may also comprise an administration once every 2, 3, 4, 5, 6, 7, 8, 9 or 10 year, or more than 10 years. In another particular embodiment, administration of each administration of a viral vector herein disclosed is done using a different virus for each successive administration, thereby avoiding a reduction of efficacy because of a possible immune response against a previously administered viral vector. For example, a first administration of a viral vector comprising an AAV8 capsid may be done, followed by the administration of a vector comprising an AAV9 capsid, or even by the administration of a virus unrelated to AAVs, such as a retroviral or lentiviral vector.

According to the present invention, a treatment may include curative, alleviation or prophylactic effects. Accordingly, therapeutic and prophylactic treatment includes amelioration of the symptoms of a particular glycogen storage disease or preventing or otherwise reducing the risk of developing a particular glycogen storage disease. The term "prophylactic" may be considered as reducing the severity or the onset of a particular condition. "Prophylactic" also includes preventing reoccurrence of a particular condition in a patient previously diagnosed with the condition. "Therapeutic" may also reduce the severity of an existing condition. The term 'treatment' is used herein to refer to any regimen that can benefit an animal, in particular a mammal, more particularly a human subject.

It is also disclosed an *ex vivo* gene therapy method for the treatment of a glycogen storage disease, comprising introducing the nucleic acid molecule or the nucleic acid construct of the invention into an isolated cell of a patient in need thereof, for example an isolated hematopoietic stem cell, and introducing said cell into said patient in need thereof. In a particular embodiment of this aspect, the nucleic acid molecule or construct is introduced into the cell with a vector as defined above. In a particular embodiment, the vector is an integrative viral vector. In a further particular embodiment, the viral vector is a retroviral vector, such as a lenviral vector. For example, a lentiviral vector as disclosed in van Til et al., 2010, Blood, 115(26), p. 5329, may be used in the practice in the method of the present disclosure.

The invention also relates to the nucleic acid molecule, the nucleic acid construct, the chimeric GAA polypeptide or the cell of the invention for use as a medicament. The present disclosure also provides the vector herein disclosed, for use as a medicament.

The invention also relates to the nucleic acid molecule, the nucleic acid construct, the chimeric GAA polypeptide or the cell of the invention, for use in a method for treating a disease caused by a mutation in the GAA gene, in particular in a method for treating Pompe disease. It is also herein provided the vector as disclosed above, for use in a method for treating a disease caused by a mutation in the GAA gene, in particular in a method for treating Pompe disease. The invention further relates to the nucleic acid molecule, the nucleic acid construct, the chimeric GAA polypeptide or the cell of the invention, for use in a method for treating a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII. A vector as herein disclosed is also provided for use in a method for treating a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII. The chimeric GAA polypeptide of the invention may be administered to a patient in need thereof, for use in enzyme replacement therapy (ERT), such as for use in enzyme replacement therapy of one of a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII.

It is also disclosed the use of the nucleic acid molecule, the nucleic acid construct, the vector, the chimeric GAA polypeptide or the cell of the invention, in the manufacture of a medicament useful for treating a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII .

### EXAMPLES

The invention is further described in detail by reference to the following experimental examples and the attached figures. These examples are provided for purposes of illustration only, and are not intended to be limiting.

### MATERIAL AND METHODS

### GAA activity

GAA activity was measured following homogenization of frozen tissue samples in distilled water. 50-100 mg of tissue were weighed and homogenized, then centrifuged for 20 minutes at 10000 x g. The reaction was set up with 10 µl of supernatant and 20 µl of substrate - 4MUα-D-glucoside, in a 96 wells plate. The reaction mixture was incubated at 37°C for one hour, and then stopped by adding 150 µl of Sodium Carbonate buffer pH 10.5. A standard curve (0-2500 pmol/µl of 4MU) was used to measure released fluorescent 4MU from individual reaction mixture, using the EnSpire alpha plate reader (Perkin-Elmer) at 449 nm (Emission) and 360 nm (Excitation). The protein concentration of the clarified supernatant was quantified by BCA (Thermo Fisher Scientific). To calculate the GAA activity, released 4MU concentration was divided by the sample protein concentration and activity was reported as nmol/hour/mg protein.

### Glycogen content

Glycogen content was measured indirectly as the glucose released after total digestion by *Aspergillus Niger* amyloglucosidase of the tissue homogenates obtained as described above. The reaction was set in a 96-well plate up with 20 µl of tissue homogenate and 55 µl of distilled water. Samples were incubated for 5 min at 95°C and then cooled at 4°C. 25 µl of amyloglucosidase (diluted 1:50 in 0.1M potassium acetate pH5.5) were added to each sample. A control reaction without amyloglucosidase was also set up for each sample. Both sample and control reaction were incubated at 37°C for 90 minutes. The reaction was stopped by incubating samples for 5 min at 95°C. The glucose released was determined using the Glucose assay kit (Sigma-Aldrich) by measuring the absorbance using the EnSpire alpha plate reader (Perkin-Elmer) at 540 nm

### Plethysmography

A flow-through (0.5 L/min) plethysmograph (EMKA technologies) was used to measure the pattern of breathing in control and Gaa-/- mice. A clear Plexiglas chamber was calibrated with known airflow and pressure signals before data collection. Signals were analyzed by using the IOX2 software (EMKA technologies). The following variables were measured: breathing frequency, tidal volume and minute ventilation. Ventilation data were collected in 5-min bins. Five minutes were allowed for acclimation to the chamber. During both acclimation and data acquirement, mice were breathing normoxic air (21% O2, 79% N2).

### Mouse studies

Gaa -/- mouse was generated by targeted disruption of exon 6 and is maintained on the C57BL/6J/129X1/SvJ background (Raben N. et al 1998). Vectors were delivered via the tail vein in a volume of 0.2 ml. Serum samples were collected monthly to monitor levels of secreted hGAA. PBS-injected affected animals and wild type littermates were used as controls.

### Anti-hGAA antibody determination

Maxisorp 96 wells plates (Thermo Fisher Scientific) were coated with Myozime^{®} protein in carbonate buffer at 4°C overnight. A standard curve of rat recombinant IgG (Sigma Aldrich) was coated to the wells in seven 2-fold dilution starting from 1 µg/ml. After blocking, plasma samples were added to plates and incubated 1 hr at 37C. Detection was performed by adding to the wells 3,3',5,5'-tetramethylbenzidine substrate (BD Biosciences), and color development was measured at 450 and 570 nm (for background subtraction) on an Enspire plate reader (Perkin Elmer) after blocking the reaction with H2SO4.

### NHP study

Male Cynomolgus macaques were housed in stainless steel cages and maintained on a 12-hour light/dark cycle. All macaques had neutralizing antibody titers of <1:5 before the start of the study. A dose of2E12 vg/kg of AAV8-hAAT-sp7-Δ8-hGAAco1 was infused via the saphenous vein. Blood samples were taken 12 days before and 30 days after the injection via the femoral vein. Whole blood was collected in EDTA containing tubes and centrifuged to separate serum. Three months after vector administration all macaques were euthanized. The animals were first anesthetized with a mixture of ketamine/dexmedetomidine and then euthanized using sodium pentobarbital injected IV. Tissues were immediately collected and frozen in liquid nitrogen.

### Western blot analysis

Total homogenates were obtained from frozen muscles. Protein concentration was determined in the extracts by Pierce BCA Protein Assay (Thermo Fisher Scientific), following manufacturer's instructions. Western blot was performed with an anti hGAA antibody (Abeam) . Anti-tubulin antibody (Sigma Aldrich) was used as loading controls.

### RESULTS

In an effort to improve current gene replacement therapies for Pompe disease, we engineered the hGAA sequence to increase its secretion by exchanging wild-type signal peptide (indicated here as sp1) with different signal peptides (sp2 to 8, described in table 4) in the sequence optimized sequence of hGAA (SEQ ID NO: 13).

**Table 4**

| Signal peptide | DNA sequence | Aminoacid sequence | Sequence optimized |
|---|---|---|---|
| sp1 | atgggagtgcggcaccctccatgtagccacagactgc tggccgtgtgtgccctggtgtctctggctacagctgccc tgctg | mgvrhppcshrllavcalvsla taall | YES |
| sp2 | atgcctagctctgtgtcctggggcattctgctgctggcc ggcctgtgttgtctggtgcctgtgtctctggcc | mpssvswgilllaglcclvpvsl a | YES |
| sp3 | atgctgctgctgtctgcactgctgctgggcctggcctttt ggctactct | mlllsalllglafgys | YES |
| sp4 | atgctgctgagctttgccctgctgctgggactggccct gggctactct | mllsfalllglalgys | YES |
| sp5 | atgctgctggaacatgccctgctgctgggactggccca cggctattct | mllehalllglahgys | YES |
| sp6 | atgcctccacctagaacaggcagaggcctgctgtggc tgggcctggtgctgtctagtgtgtgtgtggccctgggc | mppprtgrgllwlglvlssvcv alg | YES |
| sp7 | atggcctttctgtggctgctgagctgttgggccctgctg ggcaccaccttcggc | maflwllscwallgttfg | YES |
| sp8 | atggccagcagactgaccctgctgacactccttctgct gctgctggccggcgatagagccagcagc | masrltlltllllllagdrass | YES |

We transfected hepatoma cells (Huh-7) with plasmids expressing GFP or wild-type hGAA (hGAA; SEQ ID NO:37) in parallel with plasmids expressing codon optimized hGAA (hGAAco) fused with signal peptides 1 to 8. 48 hours after transfection the growth medium has been analyzed for the presence of hGAA. Notably only four of the constructs bearing efficient signal peptide led to the secretion of hGAA levels significantly higher than what observed in the negative control represented by GFP-transfected cells (Figure 1A). Constructs expressing the hGAA chimeric protein carrying the signal peptides sp2, sp6, sp7, and sp8 secreted higher levels of hGAA in medium (p<0.05 vs. GFP).

We then packaged these constructs in AAV8 vectors produced by triple transfection and cesium chloride purification and we injected them in wild-type C57BL/6J mice. We then compared in vivo GAA serum levels across constructs in which the signal peptides sp 1, 2, 3, 7 and 8 (figure 1B) were used. One month after the injection of 1E12 vg/kg of AAV8 vectors expressing hGAAco we observed a significantly higher level of circulating hGAA compared to PBS injected mice. Interestingly, the level of circulating hGAA was significantly higher in mice treated with vectors expressing hGAAco fused with sp2, 7, and 8. Surprisingly, secretion levels achieved with sp2 construct in vivo were significantly lower than those measured with sp7- and sp8- engineered hGAA (Figure 1B). Taken together these data indicate that the substitution of wild-type signal peptide with signal peptides deriving from a protein efficiently secreted in the liver is an effective strategy to increase circulating level of hGAA in vivo. Moreover, the unexpected results obtained in vivo with sp7 and 8 signal peptides indicate that not all signal peptides are equally efficient in vivo, and that signal peptides sp7 and sp8 drive superior efficacy of secretion in vivo compared with sp 1 and sp2.

Those findings were then confirmed in an animal model of the disease, GAA -/- mice. This mouse model presents no residual activity of the enzyme in muscle, together with glycogen accumulation in different organs, resulting in muscular strength impairment and reduced lifespan.

To compare the effectiveness of the different vectors in the rescue of the Pompe disease phenotype in GAA-/- mice, we followed long-term the effects of the injection of 2E12 vg/kg of vectors expressing hGAAco and engineered version fused with signal peptides sp2, 7, and 8. Three months after the injections, we observed significantly increased circulating hGAA after the injection with AAV8 expressing hGAAco bearing the highly efficient signal peptides sp2, 7, and 8 (Figure 2A). Notably, hGAAco fused with sp7 signal peptide leaded to levels of hGAA in circulation significantly higher than those observed for the other two constructs. The long term follow-up in this experiment permitted us to estimate the survival of GAA -/- mice. Mice were injected at 4 months of age and then followed for six months. During this period 8/10 GAA-/- mice died in the PBS injected group whereas just 1/45 death was reported in GAA-/- animals treated with hGAAco expressing constructs and in wild-type animals. The statistical significance of this finding (figure 2B) indicates that the treatment with all hGAAco expressing vectors, independently of the secretion level, rescues the lethal phenotype observed in GAA -/- mice. Another phenotype reported for this mouse model is a decreased respiratory function. In particular, a decreased tidal volume has been reported (DeRuisseau et al PNAS 2008) and it has been demonstrated that the decrease is due to the accumulation of glycogen in the nervous system. The rescue of glycogen level in the nervous system depends on the ability of the hGAA to cross the blood-brain barrier and it has been demonstrated in other lysosomal storage disorders (Polito et al Hum. Mol. Genet. 2010, Cho et al Orph. J. of Rare Dis. 2015) that this is directly dependent from the circulating levels of the protein. We therefore evaluated the effect of long-term, high circulating level of hGAA on the tidal volume of GAA -/- mice. Three months after the injections, GAA -/- mice shown a decreased, although not significantly (p = 0.104), tidal volume whereas mice treated with sp7 shown a tidal volume very similar to those observed in WT mice (p = 0.974) (figure 2C, left). Six months after the injections, only two GAA -/- mice survived and they appear to have a less severe respiratory phenotype. Again, mice treated with sp7 hGAAco had a tidal volume similar to that observed in WT animals (p= 0.969) (figure 2C, right). Importantly, a statistically significant difference between the tidal volume measured in mice treated with sp1 and sp7 hGAAco (p = 0.041) was noted, showing a more marked improvement in sp7-GAA treated mice. Taken together these data indicate that liver transduction with an AAV8 expressing hGAAco fused with sp7 signal peptide results in superior level of hGAA in the blood with a concomitant complete phenotypical correction of respiratory function in GAA-/- mice.

We then verified if the high level of circulating hGAA rescued the glycogen accumulation in skeletal muscle. We measured hGAA activity in the quadriceps of mice injected as described above. Injection of hGAA expressing vectors leaded to an increase in hGAA activity in quadriceps to levels comparable to those observed in WT animals (figure 3A). Measurement of glycogen in quadriceps indicate that GAA -/- mice accumulate ~ 20-fold more glycogen than WT animals (p = 3.5E-6). This accumulation is reversed by the treatment with hGAA expressing vector (p<0.05 vs GAA-/-), with the sp7 that shown the lowest glycogen levels, undistinguishable from the levels of wild type animals (p = 0.898 vs. WT) (Figure 3B).

To verify that the fusion of hGAA with an efficient signal peptide improve its secretion and increase the phenotypical correction of the disease in vivo, we injected GAA -/- mice a low vector dose, and we evaluated the biochemical correction of the phenotype. Three months after the injection of 6E11 vg/kg of vectors expressing hGAAco fused with signal peptide 1, 7, and 8, we measured circulating hGAA. Notably sp 7 and 8 leaded to a three-fold increase in the secreted hGAA detectable in serum compared to PBS-treated mice (Figure 4A). We further investigated the therapeutic effects of AAV8 vectors expressing hGAAco by performing biochemical analysis of tissues from treated animals and controls. We evaluated the glycogen content in heart, diaphragm, and quadriceps of GAA -/- mice treated as described above. Notably, we observed high levels of hGAA in tissues after treatment with hGAAco expressing vectors (data not shown) that correlated with a significant reduction in glycogen content in all the tissues considered (figure 4B-D). In particular, in the heart (figure 4B) the level of glycogen measured after treatment with vectors bearing the highly efficient signal peptides sp7 and 8 were undistinguishable from those observed in non-affected wild-type animals (p = 0.983 and 0.996 vs. WT respectively). Importantly, the level observed after treatment with both the sp7 and sp8 vectors were significantly reduced compared with GAA -/- animals PBS-injected or treated with wild-type hGAAco expressing vector (noted as sp1).

We also tested if the liver transduction with our vectors induced a humoral response against the transgene. Mice were injected intravenously with AAV8 vectors expressing hGAAco1 with native sp1 signal peptide (co) or Δ8-hGAAco1 fused with sp2, sp7, or sp8 under the transcriptional control of a liver specific promoter. The results are presented in Figure 5. Gaa-/- injected intramuscularly with an AAV expressing Δ8-hGAAco1 under the transcriptional control of a constitutive promoter showed very high level of total IgG (~150 µg/mL), whereas in general vector expressing the same protein in the liver showed lower level of humoral response. Interestingly, mice injected with sp1 hGAAco1 (co) expressing vector showed detectable level of antibodies at both doses, whereas mice injected with the engineered high secreted vectors had undetectable IgG levels. These data indicate that the expression of a transgene in the liver is fundamental for the induction of peripheral tolerance, also they provide indications that high circulating levels of a hGAA, achieved by the fusion with an efficient signal peptide induce a reduction in the humoral response against the protein itself.

The best performing vector selected in the mouse study was injected in two non-human primates (NHP, Macaca Fascicularis sp.) to verify the efficacy of secretion of our vector and the uptake in muscles. We injected two monkeys with 2E12 vg/kg of AAV8-hAAT-sp7-Δ8-hGAAco1. One month after the injection we measured the levels of hGAA in the serum of the two animals by western blot using a specific anti-hGAA antibody. We observed a clear band with a size compatible with that of hGAA in the two monkeys. This band was not present in serum samples obtained 12 days before vector injection, thus confirming the specificity of our detection method (Figure 6A). Three months after the injection we sacrificed the animals and we obtained tissues to verify if hGAA secreted from the liver in the bloodstream were efficiently taken up by muscle. We performed a western blot using an antibody specific for hGAA on total lysates obtained from biceps and diaphragm of the two monkeys. Interestingly we were able to observe a clear band in animal number 2 which also showed the highest levels of hGAA in the bloodstream (Figure 6B). Also, in animal number 1 we could observe a fainter band with a molecular weight consistent with that of hGAA in both muscles analyzed. These data indicate that the AAV8-hAAT-sp7-Δ8-hGAAco1 vector efficiently transduces liver in NHP. They also demonstrate that the protein secreted in the bloodstream is efficiently taken up in muscle and that this uptake is correlated with the level of hGAA measured in blood.

We further performed the analysis of GAA activity in media and lysates of HuH7 cells transfected with different GAA versions (all codon optimized): 1. native GAA including the native sp1 GAA signal peptide (co), 2. engineered GAA containing the heterologous sp7 or sp8 signal peptide (sp7-co, sp8-co). The analysis showed (figure 7) significantly higher GAA activity in media of cells transfected with engineered versions compared to native GAA (co). Interestingly, intracellular GAA activity was instead significantly higher when using native GAA (co) compared to the engineered versions, indicating that the native GAA is mainly retained in the cells.

We also determined the effect of the best performing vector selected in the mouse study (AAV8-hAAT-sp7-Δ8-hGAAco1) in a mouse model of GSDIII. We developed a knock-out mouse model for the glycogen debranching enzyme (GDE). This model recapitulates the phenotype of the disease observed in humans affected by type III glycogen storage disease (GSDIII). In particular GDE -/- mice, that completely lacks the GDE activity, have an impairment in muscle strength and accumulate glycogen in different tissues. Interestingly they also accumulate glycogen in the liver, which also is seen in humans. Here we tested if the overexpression of sp7-Δ8-hGAA in the liver rescue the glycogen accumulation observed in GDE -/- mice. We injected GDE-/- mice with 1E11 or 1E12 vg/mouse of AAV8-hAAT-sp7-Δ8-hGAAco1. As controls, we injected in parallel wild-type (WT) and GDE -/- mice with PBS. Three months after the vector administration, mice were sacrificed and the level of glycogen in the liver has been quantified. The results are reported in Figure 8. As already reported (Pagliarani et al and our model), GDE -/- mice shown a significant increase in glycogen accumulation in the liver (p=1.3E-7) with 5 times more glycogen when compared to wild-type animals. Surprisingly, the treatment with 1E11 and 1E12 vg/mouse of the AAV8-hAAT-sp7-Δ8-hGAAco1 vector induced a statistically significant decrease in the glycogen content (p=4.5E-5 and 1.4E-6 respectively). Importantly, the levels of glycogen measured in the liver of mice injected with AAV8-hAAT-sp7-Δ8-hGAAco1 vector were undistinguishable from those measured in wild-type animals in particular at the highest dose (p= 0.053 for the 1E11 dose cohort and 0.244 for the 1E12 dose cohort).

We performed the analysis of GAA activity in media and lysates of HuH7 cells transfected with different GAA versions (all codon-optimized): 1. native GAA including the native sp1 GAA signal peptide (co), 2. engineered GAA containing the heterologous sp7 signal peptide (sp7-co), and 3. engineered GAA containing the heterologous sp7 signal peptide followed by the deletion of a variable number of aminoacids (sp7-Δ8-co, sp7-Δ29-co, sp7-Δ42-co, sp7-Δ43-co, sp7-Δ47-co and sp7-Δ62-co, wherein the 8, 29, 42, 47 and 62 first N-terminal amino acids of SEQ ID NO:5 are deleted, respectively). The analysis showed (figure 9) significantly higher GAA activity in media of cells transfected with Δ8, Δ29, Δ42 and Δ43 GAA versions compared to both engineered non-deleted GAA (sp7-co) and native GAA (co). Significantly lower GAA activity was instead observed in media of cells transfected with Δ47 and Δ62 GAA versions compared to the other engineered GAA versions [deleted (sp7-Δ8-co, sp7-Δ29-co, sp7-Δ42-co, sp7-Δ43-co) and non-deleted (sp7-co)]. Interestingly, (figure 10) intracellular GAA activity was not different among the productive deletions (sp7-Δ8-co, sp7-Δ29-co, sp7-Δ42-co, sp7-Δ43-co) and the non-deleted version (sp7-co) indicating that they are all efficiently produced and processed within the cell. Intracellular GAA activity was instead very low for sp7-Δ47-co and sp7-Δ62-co versions and significantly lower when compared to all the other engineered versions [deleted (sp7-Δ8-co, sp7-Δ29-co, sp7-Δ42-co, sp7-Δ43-co) and non-deleted (sp7-co)].

We also performed the analysis of GAA activity in media and lysates of HuH7 cells transfected with different GAA versions (all codon optimized): 1. native GAA including the native sp1 GAA signal peptide (co), 2. engineered GAA containing the heterologous sp6 or sp8 signal peptide (sp6-co, sp8-co), and 3. engineered GAA containing the heterologous sp6 or sp8 signal peptide followed by the deletion of 8 amino acids (sp6-Δ8-co, sp8-Δ8-co). The analysis showed (figure 11) significantly higher GAA activity in media of cells transfected with Δ8 versions compared to: i. their respective engineered non-deleted GAA versions (sp6-co or sp8-co); and ii. native GAA (co). Interestingly, intracellular GAA activity was not different among all the engineered GAA versions (both deleted and non-deleted) indicating that they are all efficiently produced and processed within the cell (cell lysates panel). Intracellular GAA activity was instead significantly higher when using native GAA (co) compared to the engineered versions, indicating that the native GAA is mainly retained in the cell.

## Claims

1. A nucleic acid molecule encoding a functional chimeric acid alpha-glucosidase (GAA) protein, comprising a signal peptide moiety and a functional human GAA moiety,
wherein said signal peptide moiety has an amino acid sequence selected from the group consisting of SEQ ID NO:2 to 4,
or wherein said signal peptide moiety is a functional signal peptide moiety having an amino acid sequence comprising from 1 to 5 amino acid deletion(s), insertion(s) or substitution(s) as compared to the sequence of SEQ ID NOs:2 to 4.

2. The nucleic acid molecule according to claim 1, wherein said signal peptide moiety has an amino acid sequence consisting of SEQ ID NO: 2.

3. The nucleic acid molecule according to claim 1 or 2, wherein said functional human GAA moiety has 1 to 75 consecutive amino acids truncated at its N-terminal end as compared to a functional parent human GAA polypeptide.

4. The nucleic acid molecule according to any one of claims 1 to 3, wherein said functional human GAA moiety has 6, 7, 8, 9, 10, 40, 41, 42, 43, 44, 45 or 46 consecutive amino acids truncated at its N-terminal end as compared to a functional parent human GAA polypeptide.

5. The nucleic acid molecule according to any one of claims 1 to 4, wherein said functional human GAA moiety has 8, 42 or 43 consecutive amino acids truncated at its N-terminal end as compared to a functional parent human GAA polypeptide.

6. The nucleic acid molecule according to any one of claims 1 to 5, wherein said functional human GAA moiety has 8 consecutive amino acids truncated at its N-terminal end as compared to a functional parent human GAA.

7. The nucleic acid molecule according to any one of claims 3 to 6, wherein said functional parent human GAA is the human GAA polypeptide shown in SEQ ID NO:5 or SEQ ID NO:36.

8. The nucleic acid molecule according to claim 7, wherein said functional parent human GAA is the human GAA polypeptide shown in SEQ ID NO:5.

9. The nucleic acid molecule according to any one of claims 1 to 8, which is a nucleotide sequence optimized to improve the expression of and/or improve immune tolerance to said functional chimeric GAA protein *in vivo.*

10. The nucleic acid molecule according to claim 9, which is the nucleotide sequence shown in SEQ ID NO:13 or 14.

11. The nucleic acid molecule according to any one of claims 1 to 10, comprising a nucleotide sequence resulting from the combination of the following sequences:
| Signal peptide moiety coding sequence | GAA moiety coding sequence |
|---|---|
| SEQ ID NO:26 | SEQ ID NO:31 |
| SEQ ID NO:27 | |
| SEQ ID NO:28 | |
| SEQ ID NO:26 | SEQ ID NO:13 |
| SEQ ID NO:27 | |
| SEQ ID NO:28 | |
| SEQ ID NO:26 | SEQ ID NO:14 |
| SEQ ID NO:27 | |
| SEQ ID NO:28 | |
| SEQ ID NO:26 | SEQ ID NO:32 |
| SEQ ID NO:27 | |
| SEQ ID NO:28 | |
| SEQ ID NO:26 | SEQ ID NO:33 |
| SEQ ID NO:27 | |
| SEQ ID NO:28 | |
| SEQ ID NO:26 | SEQ ID NO:34 |
| SEQ ID NO:27 | |
| SEQ ID NO:28 | |
| SEQ ID NO:26 | SEQ ID NO:35 |
| SEQ ID NO:27 | |
| SEQ ID NO:28 | |
| SEQ ID NO:26 | SEQ ID NO:44 |
| SEQ ID NO:27 | |
| SEQ ID NO:28 | |
| SEQ ID NO:26 | SEQ ID NO:45 |
| SEQ ID NO:27 | |
| SEQ ID NO:28 | |
| SEQ ID NO:26 | SEQ ID NO:46 |
| SEQ ID NO:27 | |
| SEQ ID NO:28 | |
| SEQ ID NO:26 | SEQ ID NO:47 |
| SEQ ID NO:27 | |
| SEQ ID NO:28 | |
| SEQ ID NO:26 | SEQ ID NO:48 |
| SEQ ID NO:27 | |
| SEQ ID NO:28 | |
| SEQ ID NO:26 | SEQ ID NO:49 |
| SEQ ID NO:27 | |
| SEQ ID NO:28 | |
| SEQ ID NO:26 | SEQ ID NO:50 |
| SEQ ID NO:27 | |
| SEQ ID NO:28 | |
| SEQ ID NO:26 | SEQ ID NO:51 |
| SEQ ID NO:27 | |
| SEQ ID NO:28 | |
| SEQ ID NO:26 | SEQ ID NO:52 |
| SEQ ID NO:27 | |
| SEQ ID NO:28 | |
| SEQ ID NO:26 | SEQ ID NO:53 |
| SEQ ID NO:27 | |
| SEQ ID NO:28 | |
| SEQ ID NO:26 | SEQ ID NO:54 |
| SEQ ID NO:27 | |
| SEQ ID NO:28 | |

12. A nucleic acid construct, comprising the nucleic acid molecule according to any one of claims 1 to 11, which is an expression cassette comprising said nucleic acid molecule operably linked to a liver-specific promoter selected from the group consisting of the alpha-1 antitrypsin promoter (hAAT), the transthyretin promoter, the albumin promoter and the thyroxine-binding globulin (TBG) promoter, wherein said nucleic acid construct optionally further comprises an intron selected from the group consisting of a human beta globin b2 (HBB2) intron, a factor IX (FIX) intron, a chicken beta-globin intron and a SV40 intron, wherein said intron is optionally a modified HBB2 intron of SEQ ID NO:7, a modified FIX intron of SEQ ID NO:9, or a modified chicken beta-globin intron of SEQ ID NO:11.

13. The nucleic acid construct according to claim 12, comprising in this order: an enhancer; an intron; a liver-specific promoter; said nucleic acid molecule encoding said functional chimeric GAA protein; and a polyadenylation signal, said construct comprising in this order: an ApoE control region; a modified HBB2 intron; a hAAT promoter; said nucleic acid molecule encoding said functional chimeric GAA protein; and a bovine growth hormone polyadenylation signal.

14. The nucleic acid construct according to claim 13, said nucleic acid construct comprising the nucleotide sequence of SEQ ID NO:20, 21 or 22 or a nucleotide sequence resulting from any one of the combinations shown in claim 11.

15. A cell transformed with the nucleic acid molecule of any one of claims 1 to 11, or the nucleic acid construct of any one of claims 12 to 14 , wherein said cell is a liver cell or a muscle cell.

16. A chimeric GAA polypeptide, comprising a signal peptide moiety and a functional human GAA moiety, wherein said signal peptide moiety is selected from the group consisting of SEQ ID NO:2 to 4.

17. The chimeric GAA polypeptide according to claim 16, wherein said signal peptide moiety has an amino acid sequence consisting of SEQ ID NO: 2.

18. The chimeric GAA polypeptide according to claim 16 or 17, wherein said functional human GAA moiety has 8, 29, 42 or 43 consecutive amino acids truncated at its N-terminal end as compared to a functional parent human GAA polypeptide.

19. The chimeric GAA polypeptide according to any one of claims 16 to 18, comprising an amino acid sequence resulting from the combination of the following sequences:
| Signal peptide moiety | GAA moiety |
|---|---|
| SEQ ID NO:2 | SEQ ID NO:5 or SEQ ID NO:36 |
| SEQ ID NO:3 | |
| SEQ ID NO:4 | |
| SEQ ID NO:2 | SEQ ID NO:29 |
| SEQ ID NO:3 | |
| SEQ ID NO:4 | |
| SEQ ID NO:2 | SEQ ID NO:41 |
| SEQ ID NO:3 | |
| SEQ ID NO:4 | |
| SEQ ID NO:2 | SEQ ID NO:30 |
| SEQ ID NO:3 | |
| SEQ ID NO:4 | |
| SEQ ID NO:2 | SEQ ID NO:42 |
| SEQ ID NO:3 | |
| SEQ ID NO:4 | |
| SEQ ID NO:2 | SEQ ID NO:43 |
| SEQ ID NO:3 | |
| SEQ ID NO:4 | |

20. A pharmaceutical composition, comprising, in a pharmaceutically acceptable carrier, the nucleic acid molecule of any one of claims 1 to 11, the nucleic acid construct of any one of claims 12 to 14, the cell according to claim 15, or the chimeric GAA polypeptide according to any of claims 16 to 19.

21. The nucleic acid molecule of any of claims 1 to 11, the nucleic acid construct of any one of claims 12 to 14, the cell according to claim 15, or the chimeric GAA polypeptide according to any one of claims 16 to 19, for use as a medicament.

22. The nucleic acid molecule of any one of claims 1 to 11, the nucleic acid construct of any one of claims 12 to 14, the cell according to claim 15, or the chimeric GAA polypeptide according to any of claims 16 to 19, for use in a method for treating a glycogen storage disease selected from the group consisting of GSDI (von Gierke's disease), GSDII (Pompe disease), GSDIII (Cori disease), GSDIV, GSDV, GSDVI, GSDVII, GSDVIII and lethal congenital glycogen storage disease of the heart.

## Patentansprüche

1. Ein Nukleinsäuremolekül, das ein funktionelles chimäres Säure-alpha-Glucosidase (GAA)-Protein kodiert, das einen Signalpeptidteil und einen funktionellen menschlichen GAA-Teil umfasst,
wobei der Signalpeptidteil eine Aminosäuresequenz aufweist, die aus der Gruppe bestehend aus SEQ ID NO:2 bis 4 ausgewählt ist,
oder wobei der Signalpeptidteil ein funktioneller Signalpeptidteil mit einer Aminosäuresequenz ist, die 1 bis 5 Aminosäuredeletion(en), -insertion(en) oder - substitution(en) im Vergleich zu der Sequenz nach SEQ ID NO:2 bis 4 umfasst.

2. Das Nukleinsäuremolekül nach Anspruch 1, wobei der Signalpeptidteil eine Aminosäuresequenz aufweist, die aus SEQ ID NO: 2 besteht.

3. Das Nukleinsäuremolekül nach Anspruch 1 oder 2, wobei der funktionelle menschliche GAA-Teil an seinem N-terminalen Ende im Vergleich zu einem funktionellen menschlichen Ausgangs-GAA-Polypeptid um 1 bis 75 aufeinanderfolgende Aminosäuren verkürzt ist.

4. Das Nukleinsäuremolekül nach einem der Ansprüche 1 bis 3, wobei der funktionelle menschliche GAA-Teil an seinem N-terminalen Ende im Vergleich zu einem funktionellen menschlichen Ausgangs-GAA-Polypeptid um 6, 7, 8, 9, 10, 40, 41, 42, 43, 44, 45 oder 46 aufeinanderfolgende Aminosäuren verkürzt ist.

5. Das Nukleinsäuremolekül nach einem der Ansprüche 1 bis 4, wobei der funktionelle menschliche GAA-Teil an seinem N-terminalen Ende im Vergleich zu einem funktionellen menschlichen Ausgangs-GAA-Polypeptid um 8, 42 oder 43 aufeinanderfolgende Aminosäuren verkürzt ist.

6. Das Nukleinsäuremolekül nach einem der Ansprüche 1 bis 5, wobei der funktionelle menschliche GAA-Teil an seinem N-terminalen Ende im Vergleich zu einem funktionellen menschlichen Ausgangs-GAA-Polypeptid um 8 aufeinanderfolgende Aminosäuren verkürzt ist.

7. Das Nukleinsäuremolekül nach einem der Ansprüche 3 bis 6, wobei das funktionelle menschliche Ausgangs-GAA das in SEQ ID NO:5 oder SEQ ID NO:36 gezeigte menschliche GAA-Polypeptid ist.

8. Das Nukleinsäuremolekül nach Anspruch 7, wobei das funktionelle menschliche Ausgangs-GAA das in SEQ ID NO:5 gezeigte menschliche GAA-Polypeptid ist.

9. Das Nukleinsäuremolekül nach einem der Ansprüche 1 bis 8, bei dem es sich um eine Nukleotidsequenz handelt, die optimiert ist um die Expression und/oder die Immuntoleranz gegenüber dem funktionellen chimären GAA-Proteins *in vivo* zu verbessern.

10. Das Nukleinsäuremolekül nach Anspruch 9, bei dem es sich um die in SEQ ID NO:13 oder 14 gezeigte Nukleotidsequenz handelt.

11. Das Nukleinsäuremolekül nach einem der Ansprüche 1 bis 10, umfassend eine Nukleotidsequenz, die sich aus der Kombination der folgenden Sequenzen ergibt:
| Kodierende Sequenz für Signalpeptidteil | Kodierende Sequenz für GAA-Teil |
|---|---|
| SEQ ID NO: 26 | SEQ ID NO: 31 |
| SEQ ID NO: 27 | |
| SEQ ID NO: 28 | |
| SEQ ID NO: 26 | SEQ ID NO: 13 |
| SEQ ID NO: 27 | |
| SEQ ID NO: 28 | |
| SEQ ID NO: 26 | SEQ ID NO: 14 |
| SEQ ID NO: 27 | |
| SEQ ID NO: 28 | |
| SEQ ID NO: 26 | SEQ ID NO: 32 |
| SEQ ID NO: 27 | |
| SEQ ID NO: 28 | |
| SEQ ID NO: 26 | SEQ ID NO: 33 |
| SEQ ID NO: 27 | |
| SEQ ID NO: 28 | |
| SEQ ID NO: 26 | SEQ ID NO: 34 |
| SEQ ID NO: 27 | |
| SEQ ID NO: 28 | |
| SEQ ID NO: 26 | SEQ ID NO: 35 |
| SEQ ID NO: 27 | |
| SEQ ID NO: 28 | |
| SEQ ID NO: 26 | SEQ ID NO: 44 |
| SEQ ID NO: 27 | |
| SEQ ID NO: 28 | |
| SEQ ID NO: 26 | SEQ ID NO: 45 |
| SEQ ID NO: 27 | |
| SEQ ID NO: 28 | |
| SEQ ID NO: 26 | SEQ ID NO: 46 |
| SEQ ID NO: 27 | |
| SEQ ID NO: 28 | |
| SEQ ID NO: 26 | SEQ ID NO: 47 |
| SEQ ID NO: 27 | |
| SEQ ID NO: 28 | |
| SEQ ID NO: 26 | SEQ ID NO: 48 |
| SEQ ID NO: 27 | |
| SEQ ID NO: 28 | |
| SEQ ID NO: 26 | SEQ ID NO: 49 |
| SEQ ID NO: 27 | |
| SEQ ID NO: 28 | |
| SEQ ID NO: 26 | SEQ ID NO: 50 |
| SEQ ID NO: 27 | |
| SEQ ID NO: 28 | |
| SEQ ID NO: 26 | SEQ ID NO: 51 |
| SEQ ID NO: 27 | |
| SEQ ID NO: 28 | |
| SEQ ID NO: 26 | SEQ ID NO: 52 |
| SEQ ID NO: 27 | |
| SEQ ID NO: 28 | |
| SEQ ID NO: 26 | SEQ ID NO: 53 |
| SEQ ID NO: 27 | |
| SEQ ID NO: 28 | |
| SEQ ID NO: 26 | SEQ ID NO: 54 |
| SEQ ID NO: 27 | |
| SEQ ID NO: 28 | |

12. Ein Nukleinsäurekonstrukt, umfassend das Nukleinsäuremolekül nach einem der Ansprüche 1 bis 11, das eine Expressionskassette ist, die das Nukleinsäuremolekül umfasst, das funktionsfähig mit einem leberspezifischen Promotor verknüpft ist, der aus der Gruppe bestehend aus dem alpha-l-Antitrypsin-Promotor (hAAT), dem Transthyretin-Promotor, dem Albumin-Promotor und dem Thyroxin-bindenden Globulin (TBG)-Promotor ausgewählt ist, wobei das Nukleinsäurekonstrukt gegebenenfalls ferner ein Intron umfasst, das aus der Gruppe bestehend aus einem menschlichen Beta-Globin b2 (HBB2)-Intron, einem Faktor IX (FIX)-Intron, einem Hühner-Beta-Globin-Intron und einem SV40-Intron ausgewählt ist, wobei das Intron gegebenenfalls ein modifiziertes HBB2-Intron nach SEQ ID NO:7, ein modifiziertes FIX-Intron nach SEQ ID NO:9 oder ein modifiziertes Hühner-Beta-Globin-Intron nach SEQ ID NO:11 ist.

13. Das Nukleinsäurekonstrukt nach Anspruch 12, umfassend in dieser Reihenfolge: einen Enhancer; ein Intron; einen leberspezifischen Promotor; das Nukleinsäuremolekül, das das funktionelle chimäre GAA-Protein kodiert; und ein Polyadenylierungssignal, wobei das Konstrukt in dieser Reihenfolge umfasst: eine ApoE-Kontrollregion; ein modifiziertes HBB2-Intron; einen hAAT-Promotor; das Nukleinsäuremolekül, das für das funktionelle chimäre GAA-Protein kodiert; und ein Rinderwachstumshormon-Polyadenylierungssignal.

14. Das Nukleinsäurekonstrukt nach Anspruch 13, wobei das Nukleinsäurekonstrukt die Nukleotidsequenz nach SEQ ID NO:20, 21 oder 22 oder eine Nukleotidsequenz umfasst, die aus einer der in Anspruch 11 gezeigten Kombinationen resultiert.

15. Eine Zelle, die mit dem Nukleinsäuremolekül nach einem der Ansprüche 1 bis 11 oder dem Nukleinsäurekonstrukt nach einem der Ansprüche 12 bis 14 transformiert ist, wobei die Zelle eine Leberzelle oder eine Muskelzelle ist.

16. Ein chimäres GAA-Polypeptid, umfassend einen Signalpeptidteil und einen funktionellen menschlichen GAA-Teil, wobei der Signalpeptidteil ausgewählt ist aus der Gruppe bestehend aus SEQ ID NO:2 bis 4.

17. Das chimäres GAA-Polypeptid nach Anspruch 16, wobei der Signalpeptidteil eine Aminosäuresequenz aufweist, die aus SEQ ID NO: 2 besteht.

18. Das chimäres GAA-Polypeptid nach Anspruch 16 oder 17, wobei der funktionelle menschliche GAA-Teil an seinem N-terminalen Ende im Vergleich zu einem funktionellen menschlichen Ausgangs-GAA-Polypeptid um 8, 29, 42 oder 43 aufeinanderfolgende Aminosäuren verkürzt ist.

19. Das chimäres GAA-Polypeptid nach einem der Ansprüche 16 bis 18, umfassend eine Aminosäuresequenz, die sich aus der Kombination der folgenden Sequenzen ergibt:
| Signalpeptid-Teil | GAA-Teil |
|---|---|
| SEQ ID NO: 2 | SEQ ID NO: 5 oder SEQ ID NO: 36 |
| SEQ ID NO: 3 | |
| SEQ ID NO: 4 | |
| SEQ ID NO: 2 | SEQ ID NO: 29 |
| SEQ ID NO: 3 | |
| SEQ ID NO: 4 | |
| SEQ ID NO: 2 | SEQ ID NO: 41 |
| SEQ ID NO: 3 | |
| SEQ ID NO: 4 | |
| SEQ ID NO: 2 | SEQ ID NO: 30 |
| SEQ ID NO: 3 | |
| SEQ ID NO: 4 | |
| SEQ ID NO: 2 | SEQ ID NO: 42 |
| SEQ ID NO: 3 | |
| SEQ ID NO: 4 | |
| SEQ ID NO: 2 | SEQ ID NO: 43 |
| SEQ ID NO: 3 | |
| SEQ ID NO: 4 | |

20. Eine pharmazeutische Zusammensetzung, die in einem pharmazeutisch akzeptablen Träger das Nukleinsäuremolekül nach einem der Ansprüche 1 bis 11, das Nukleinsäurekonstrukt nach einem der Ansprüche 12 bis 14, die Zelle nach Anspruch 15 oder das chimäre GAA-Polypeptid nach einem der Ansprüche 16 bis 19 enthält.

21. Das Nukleinsäuremolekül nach einem der Ansprüche 1 bis 11, das Nukleinsäurekonstrukt nach einem der Ansprüche 12 bis 14, die Zelle nach Anspruch 15 oder das chimäre GAA-Polypeptid nach einem der Ansprüche 16 bis 19, zur Verwendung als Medikament.

22. Das Nukleinsäuremolekül nach einem der Ansprüche 1 bis 11, das Nukleinsäurekonstrukt nach einem der Ansprüche 12 bis 14, die Zelle nach Anspruch 15 oder das chimäre GAA-Polypeptid nach einem der Ansprüche 16 bis 19, zur Verwendung in einem Verfahren zur Behandlung einer Glykogenspeicherkrankheit, ausgewählt aus der Gruppe bestehend aus GSDI (von Gierke-Krankheit), GSDII (Pompe-Krankheit), GSDIII (Cori-Krankheit), GSDIV, GSDV, GSDVI, GSDVII, GSDVIII und tödlicher angeborener Glykogenspeicherkrankheit des Herzens.

## Revendications

1. Molécule d'acide nucléique codant une protéine alpha-glucosidase acide (GAA) chimérique fonctionnelle, comprenant un fragment de peptide signal et un fragment de GAA humaine fonctionnelle,
dans laquelle ledit fragment de peptide signal a une séquence d'acides aminés choisie dans le groupe constitué par les SEQ ID NO : 2 à 4,
ou dans laquelle ledit fragment de peptide signal est un fragment de peptide signal fonctionnel ayant une séquence d'acides aminés comprenant 1 à 5 délétions, insertions ou substitutions d'acides aminés en comparaison avec la séquence des SEQ ID NO : 2 à 4.

2. Molécule d'acide nucléique selon la revendication 1, dans laquelle ledit fragment de peptide signal a une séquence d'acides aminés consistant en la SEQ ID NO : 2.

3. Molécule d'acide nucléique selon la revendication 1 ou 2, dans laquelle ledit fragment de GAA humaine fonctionnelle a 1 à 75 acides aminés consécutifs tronqués à son extrémité N-terminale en comparaison avec un polypeptide de GAA humaine parente fonctionnelle.

4. Molécule d'acide nucléique selon l'une quelconque des revendications 1 à 3, dans laquelle ledit fragment de GAA humaine fonctionnelle a 6, 7, 8, 9, 10, 40, 41, 42, 43, 44, 45 ou 46 acides aminés consécutifs tronqués à son extrémité N-terminale en comparaison avec un polypeptide de GAA humaine parente fonctionnelle.

5. Molécule d'acide nucléique selon l'une quelconque des revendications 1 à 4, dans laquelle ledit fragment de GAA humaine fonctionnelle a 8, 42 ou 43 acides aminés consécutifs tronqués à son extrémité N-terminale en comparaison avec un polypeptide de GAA humaine parente fonctionnelle.

6. Molécule d'acide nucléique selon l'une quelconque des revendications 1 à 5, dans laquelle ledit fragment de GAA humaine fonctionnelle a 8 acides aminés consécutifs tronqués à son extrémité N-terminale en comparaison avec un polypeptide de GAA humaine parente fonctionnelle.

7. Molécule d'acide nucléique selon l'une quelconque des revendications 3 à 6, dans laquelle ladite GAA humaine parente fonctionnelle est le polypeptide de GAA représenté dans la SEQ ID NO : 5 ou la SEQ ID NO : 36.

8. Molécule d'acide nucléique selon la revendication 7, dans laquelle ladite GAA humaine parente fonctionnelle est le polypeptide de GAA humaine représenté dans la SEQ ID NO : 5.

9. Molécule d'acide nucléique selon l'une quelconque des revendications 1 à 8, qui a une séquence de nucléotides optimisée pour améliorer l'expression et/ou améliorer la tolérance immunitaire à ladite protéine GAA chimérique fonctionnelle *in vivo.*

10. Molécule d'acide nucléique selon la revendication 9, qui est la séquence de nucléotides représentée dans la SEQ ID NO : 13 ou 14.

11. Molécule d'acide nucléique selon l'une quelconque des revendications 1 à 10, comprenant une séquence de nucléotides résultant de la combinaison des séquences suivantes :
| Séquence codante de fragment de peptide signal | Séquence codante de fragment de GAA |
|---|---|
| SEQ ID NO : 26 | SEQ ID NO : 31 |
| SEQ ID NO : 27 | |
| SEQ ID NO : 28 | |
| SEQ ID NO : 26 | SEQ ID NO : 13 |
| SEQ ID NO : 27 | |
| SEQ ID NO : 28 | |
| SEQ ID NO : 26 | SEQ ID NO : 14 |
| SEQ ID NO : 27 | |
| SEQ ID NO : 28 | |
| SEQ ID NO : 26 | SEQ ID NO : 32 |
| SEQ ID NO : 27 | |
| SEQ ID NO : 28 | |
| SEQ ID NO : 26 | SEQ ID NO : 33 |
| SEQ ID NO : 27 | |
| SEQ ID NO : 28 | |
| SEQ ID NO : 26 | SEQ ID NO : 34 |
| SEQ ID NO : 27 | |
| SEQ ID NO : 28 | |
| SEQ ID NO : 26 | SEQ ID NO : 35 |
| SEQ ID NO : 27 | |
| SEQ ID NO : 28 | |
| SEQ ID NO : 26 | SEQ ID NO : 44 |
| SEQ ID NO : 27 | |
| SEQ ID NO : 28 | |
| SEQ ID NO : 26 | SEQ ID NO : 45 |
| SEQ ID NO : 27 | |
| SEQ ID NO : 28 | |
| SEQ ID NO : 26 | SEQ ID NO : 46 |
| SEQ ID NO : 27 | |
| SEQ ID NO : 28 | |
| SEQ ID NO : 26 | SEQ ID NO : 47 |
| SEQ ID NO : 27 | |
| SEQ ID NO : 28 | |
| SEQ ID NO : 26 | SEQ ID NO : 48 |
| SEQ ID NO : 27 | |
| SEQ ID NO : 28 | |
| SEQ ID NO : 26 | SEQ ID NO : 49 |
| SEQ ID NO : 27 | |
| SEQ ID NO : 28 | |
| SEQ ID NO : 26 | SEQ ID NO : 50 |
| SEQ ID NO : 27 | |
| SEQ ID NO : 28 | |
| SEQ ID NO : 26 | SEQ ID NO : 51 |
| SEQ ID NO : 27 | |
| SEQ ID NO : 28 | |
| SEQ ID NO : 26 | SEQ ID NO : 52 |
| SEQ ID NO : 27 | |
| SEQ ID NO : 28 | |
| SEQ ID NO : 26 | SEQ ID NO : 53 |
| SEQ ID NO : 27 | |
| SEQ ID NO : 28 | |
| SEQ ID NO : 26 | SEQ ID NO : 54 |
| SEQ ID NO : 27 | |
| SEQ ID NO : 28 | |

12. Construction d'acide nucléique, comprenant la molécule d'acide nucléique de l'une quelconque des revendications 1 à 11, qui est une cassette d'expression comprenant ladite molécule d'acide nucléique liée de manière fonctionnelle à un promoteur spécifique du foie choisi dans le groupe constitué par le promoteur d'antitrypsine alpha-1 (hAAT), le promoteur de transthyrétine, le promoteur d'albumine et le promoteur de globuline se liant à la thyroxine (TBG), laquelle construction d'acide nucléique comprend éventuellement en outre un intron choisi dans le groupe constitué par un intron de bêta-globine b2 humaine (HBB2), un intron de facteur IX (FIX), un intron de bêta-globine de poulet et un intron de SV40, dans laquelle ledit intron est éventuellement un intron de HBB2 modifié de la SEQ ID NO : 7, un intron de FIX modifié de la SEQ ID NO : 9, ou un intron de bêta-globine de poulet modifié de la SEQ ID NO : 11.

13. Construction d'acide nucléique selon la revendication 12, comprenant, dans cet ordre : un amplificateur ; un intron ; un promoteur spécifique du foie ; ladite molécule d'acide nucléique codant ladite protéine GAA chimérique fonctionnelle ; et un signal de polyadénylation, ladite construction comprenant dans cet ordre : une région de contrôle d'ApoE ; un intron de HBB2 modifié ; un promoteur de hAAT ; ladite molécule d'acide nucléique codant ladite protéine GAA chimérique fonctionnelle ; et un signal de polyadénylation d'hormone de croissance bovine.

14. Construction d'acide nucléique selon la revendication 13, laquelle construction d'acide nucléique comprend la séquence de nucléotides de la SEQ ID NO : 20, 21 ou 22 ou une séquence de nucléotides résultant de l'une quelconque des combinaisons indiquées dans la revendication 11.

15. Cellule transformée par la molécule d'acide nucléique de l'une quelconque des revendications 1 à 11, ou la construction d'acide nucléique de l'une quelconque des revendications 12 à 14, laquelle cellule est une cellule hépatique ou une cellule musculaire.

16. Polypeptide de GAA chimérique, comprenant un fragment de peptide signal et un fragment de GAA humaine fonctionnelle, dans lequel ledit fragment de peptide signal est choisi dans le groupe constitué par les SEQ ID NO : 2 à 4.

17. Polypeptide de GAA chimérique selon la revendication 16, dans lequel ledit fragment de peptide signal a une séquence d'acides aminés consistant en la SEQ ID NO : 2.

18. Polypeptide de GAA chimérique selon la revendication 16 ou 17, dans lequel ledit fragment de GAA humaine fonctionnelle a 8, 29, 42 ou 43 acides aminés consécutifs tronqués à son extrémité N-terminale en comparaison avec un polypeptide de GAA humaine parente fonctionnelle.

19. Polypeptide de GAA chimérique selon l'une quelconque des revendications 16 à 18, comprenant une séquence d'acides aminés résultant de la combinaison des séquences suivantes :
| Fragment de peptide signal | Fragment de GAA |
|---|---|
| SEQ ID NO : 2 | SEQ ID NO : 5 ou SEQ ID NO : 36 |
| SEQ ID NO : 3 | |
| SEQ ID NO : 4 | |
| SEQ ID NO : 2 | SEQ ID NO : 29 |
| SEQ ID NO : 3 | |
| SEQ ID NO : 4 | |
| SEQ ID NO : 2 | SEQ ID NO : 41 |
| SEQ ID NO : 3 | |
| SEQ ID NO : 4 | |
| SEQ ID NO : 2 | SEQ ID NO : 30 |
| SEQ ID NO : 3 | |
| SEQ ID NO : 4 | |
| SEQ ID NO : 2 | SEQ ID NO : 42 |
| SEQ ID NO : 3 | |
| SEQ ID NO : 4 | |
| SEQ ID NO : 2 | SEQ ID NO : 43 |
| SEQ ID NO : 3 | |
| SEQ ID NO : 4 | |

20. Composition pharmaceutique comprenant, dans un véhicule pharmaceutiquement acceptable, la molécule d'acide nucléique de l'une quelconque des revendications 1 à 11, la construction d'acide nucléique de l'une quelconque des revendications 12 à 14, la cellule de la revendication 15, ou le polypeptide de GAA chimérique de l'une quelconque des revendications 16 à 19.

21. Molécule d'acide nucléique selon l'une quelconque des revendications 1 à 11, construction d'acide nucléique selon l'une quelconque des revendications 12 à 14, cellule selon la revendication 15, ou polypeptide de GAA chimérique selon l'une quelconque des revendications 16 à 19, pour une utilisation en tant que médicament.

22. Molécule d'acide nucléique selon l'une quelconque des revendications 1 à 11, construction d'acide nucléique selon l'une quelconque des revendications 12 à 14, cellule selon la revendication 15, ou polypeptide de GAA chimérique selon l'une quelconque des revendications 16 à 19, pour une utilisation dans une méthode de traitement d'une glycogénose choisie dans le groupe constitué par la GSDI (maladie de von Gierke), la GSDII (maladie de Pompe), la GSDIII (maladie de Cori), la GSDIV, la GSDV, la GSDVI, la GSDVII, la GSDVIII et la glycogénose congénitale létale du coeur.
